# EUROPEAN PATENT APPLICATION

(11) **EP 4 653 454 A1**
(43) Date of publication of application: **26.11.2025**
(21) Application number: 23917311.5
(22) Date of filing: 21.12.2023
(51) Int. Cl.: C07K 14/165, C07K 19/00, C12N 15/50, C12N 15/62, C12N 15/63, C12N 5/10, A61K 39/215, A61K 39/295, A61P 31/14

(54) **PROTEIN AND VACCINE FOR RESISTING INFECTION FROM SARS-COV-2 OMICRON MUTANT STRAIN AND SUBTYPE THEREOF**

(30) Priority: 19.01.2023 CN 202310058902
(71) Applicant: WestVac Biopharma Co., Ltd., Chengdu, Sichuan 610000 (CN)
(72) Inventor: WEI, Xiawei, Chengdu, Sichuan 610065 (CN); LU, Guangwen, Chengdu, Sichuan 610065 (CN); YANG, Jingyun, Chengdu, Sichuan 610065 (CN); YANG, Li, Chengdu, Sichuan 610065 (CN); LI, Jiong, Chengdu, Sichuan 610065 (CN); WANG, Wei, Chengdu, Sichuan 610065 (CN); WEI, Yuquan, Chengdu, Sichuan 610065 (CN); WANG, Zhenling, Chengdu, Sichuan 610065 (CN); SHEN, Guobo, Chengdu, Sichuan 610065 (CN); ZHAO, Zhiwei, Chengdu, Sichuan 610065 (CN); YANG, Jinliang, Chengdu, Sichuan 610065 (CN)
(74) Representative: Gulde & Partner
(86) International application number: PCT/CN2023/140715
(87) International publication number: WO 2024/152844

(57) **Abstract**

The present invention relates to the field of medicines, and relates to a protein and vaccine for resisting infection from a SARS-CoV-2 Omicron mutant strain and a subtype thereof. In order to solve the problem of lack of drugs for effective prevention and treatment for infections from the SARS-CoV-2 Omicron mutant strain and the subtype thereof, the present invention provides the protein and the vaccine for resisting infection from the SARS-CoV-2 Omicron mutant strain and the subtype thereof. The vaccine is optimally designed on the basis of an RBD sequence in an S protein of the SARS-CoV-2 Omicron mutant strain and substrains BA.4/5, BQ.1.1, and XBB.1.5, can help a host to resist a coronavirus infection, and particularly has a relatively good prevention and treatment effect on a cross infection caused by a SARS-CoV-2 Omicron mutant strain and subtype viruses thereof.

## Description

### Technical Field

The present invention relates to a protein and vaccine against infection by a SARS-CoV-2 Omicron variant strain and a subtype thereof, and belongs to the medicine field.

### Background

The novel coronavirus (SARS-Cov-2) is a new β coronavirus named by the World Health Organization. This virus is enveloped, and its particles are round or oval, often polymorphic, with a diameter of 60nm-140 nm. Its genetic characteristics are significantly different from those of SARS-CoV and MERS-CoV, representing a novel coronavirus strain that has not been discovered in humans before. At present, there are five main types of SARS-Cov-2 variants: Alpha, Beta, Gamma, Delta and Omicron, and Omicron variant strains are further divided into several sublines, such as BA.1, BA.2, BA.2.12.1, BA.4, BA.5, BQ.1.1 and XBB.1.5.

Main structural proteins of SARS-CoV-2 include spike (Spike, S) protein, envelope (Envelope, E) protein, membrane (Membrane, M) protein and nucleocapsid (Nucleocapsid, N) protein, among which the S protein plays a key role in virus infection and virulence and is often used as an antigen for vaccines. Because SARS-CoV-2 variant strains carry multiple mutation sites and the S protein of the virus also carries multiple mutation sites, the variant strains can escape antibodies elicited by vaccines of SARS-CoV-2 precursor strains to some extent, thereby diminishing the efficacy of SARS-CoV-2 vaccines or decreasing its protective immunity, and posing significant challenges to epidemic control efforts. Therefore, the development of vaccines against various variants of SARS-CoV-2 virus, especially broad-spectrum vaccines against various variants of SARS-CoV-2 virus, is of great significance for the prevention and control of SARS-CoV-2.

### Summary

The present invention aims at solving at least one of the technical problems existing in the existing technology. Therefore, the objective of the present invention is to provide a protein against infection by a SARS-CoV-2 Omicron variant strain and a subtype thereof. The other objective of the present invention is to provide a vaccine containing the protein and used for preventing and/or treating infection by a SARS-CoV-2 Omicron variant strain and a subtype thereof.

The present invention provides a protein against infection by a SARS-CoV-2 Omicron variant strain and a subtype thereof, comprising an amino acid sequence as shown in any one of SEQ ID No.1, SEQ ID No.2, SEQ ID No.3, SEQ ID No.4, SEQ ID No.5 or SEQ ID No.6; or an amino acid sequence that has a homology of over 98% and the same or similar biological activity with the amino acid sequence as shown in any one of SEQ ID No.1-SEQ ID No.6.

Further, the homologous amino acid sequence as shown in SEQ ID No.1, SEQ ID No.2, or SEQ ID No.3 meets at least one of the following items: in SEQ ID No.1, SEQ ID No.2, or SEQ ID No.3, the 52nd amino acid F is mutated into S; the 54th amino acid P is mutated into S; and the 56th amino acid F is mutated into S..

Further, the homologous amino acid sequence as shown in SEQ ID No.4 or SEQ ID No.5 meets at least one of the following items: in SEQ ID No.4 or SEQ ID No.5, the 351th amino acid sequence F is mutated into S; the 353th amino acid sequence P is mutated into S; and the 355th amino acid sequence F is mutated into S; and the homologous amino acid sequence as shown in SEQ ID No.6 meets at least one of the following items: in SEQ ID No.6, the 352th amino acid sequence F is mutated into S; the 354th amino acid sequence P is mutated into S; and the 356th amino acid sequence F is mutated into S.

Furthermore, the amino acid sequence of the protein is selected from at least one of SEQ ID No.1-SEQ ID No.12.

The present invention further provides a precursor of the protein, and a signal peptide and/or a protein tag is linked to the protein against infection by the SARS-CoV-2 Omicron variant strain and the subtype thereof.

Preferably, the protein tag is selected from at least one of the following: a histidine tag (6His tag), a thioredoxin tag (Trx tag), a glutathione transferase tag, a ubiquitin-like modified protein tag, a maltose-binding protein tag, a c-Myc protein tag, an Avi protein tag, and a nitrogen source using substance A protein tag.

Further, a protease recognition region for excising the protein tag is also linked to the protein against infection by the SARS-CoV-2 Omicron variant strain and the subtype thereof.

Preferably, the protease is selected from at least one of the following: enterokinase (EK enzyme), TEV protease, thrombin, coagulation factor Xa, carboxypeptidase A and rhinovirus 3c protease. Furthermore, the amino acid sequence of the precursor is selected from at least one of SEQ ID No. 13-SEQ ID No.24.

The present invention further provides a polynucleotide, encoding the protein or the precursor. Further, the nucleotide sequence is selected from at least one of SEQ ID No.25-SEQ ID No.30. The present invention further provides a recombinant vector, comprising the polynucleotide.

Further, the recombinant vector adopts at least one of an insect baculovirus expression vector, a mammalian cell expression vector, an Escherichia coli expression vector and a yeast expression vector.

Preferably, the insect baculovirus expression vector is pFastBac1.

Preferably, the Escherichia coli expression vector is pET32a.

Preferably, the yeast expression vector is pPICZaA.

Preferably, the mammalian cell expression vector is a CHO cell expression vector.

More preferably, the CHO cell expression vector is pTT5 or FTP-002.

The present invention further provides a host cell, comprising the recombinant vector.

Further, the host cell adopts at least one of an insect cell, a mammalian cell, Escherichia coli and a yeast. Preferably, the insect cell is selected from at least one of a sf9 cell, a sf21 cell and a Hi5 cell.

Preferably, the mammalian cell is a CHO cell.

The present invention further provides a preparation method for the protein or the precursor, and the method includes the following steps: culturing the host cell to express the protein or precursor, then recovering the protein, to obtain the protein.

The present invention further provides a protein composition against infection by SARS-CoV-2 or a mutant thereof, comprising a combination of at least two or more of a Delta protein, a Delta full-length S protein, a BA.5 protein, a BQ.1.1 protein, an XBB.1.5 protein, a BA.4/5 full-length S protein, a BQ.1.1 full-length S protein, an XBB.1.5 full-length S protein, a Mu-BA.5 protein, a Mu-BQ.1.1 protein, a Mu-XBB.1.5 protein, a full-length S protein after BA.4/5 amino acid mutation, a full-length S protein after BQ.1.1 amino acid mutation and a full-length S protein after XBB.1.5 amino acid mutation; and preferably, the combinations of at least 2-4 proteins.

More preferably, the protein composition includes a combination of at least two or more of the BA.5 protein, the BQ.1.1 protein, the XBB.1.5 protein, the BA.4/5 full-length S protein, the BQ.1.1 full-length S protein and the XBB.1.5 full-length S protein.

More preferably, the protein composition includes a combination of at least two or more of the Delta protein, the Mu-BA.5 protein, the Mu-BQ.1.1 protein and the Mu-XBB.1.5 protein.

More preferably, the protein composition includes a combination of at least two or more of the Mu-BA.5 protein, the Mu-BQ.1.1 protein, the Mu-XBB.1.5 protein, the full-length S protein after the BA.4/5 amino acid mutation, the full-length S protein after the BQ.1.1 amino acid mutation and the full-length S protein after the XBB.1.5 amino acid mutation.

Further, the BA.5 protein, the BQ.1.1 protein, the XBB.1.5 protein, the BA.4/5 full-length S protein, the BQ.1.1 full-length S protein, the XBB.1.5 full-length S protein, the Mu-BA.5 protein, the Mu-BQ.1.1 protein, the Mu-XBB.1.5 protein, the full-length S protein after the BA.4/5 amino acid mutation, the full-length S protein after the BQ.1.1 amino acid mutation and the full-length S protein after the XBB.1.5 amino acid mutation includes the protein or the precursor. Further, amino acid sequences of the Delta protein, the Delta full-length S protein, the BA.5 protein, the BQ.1.1 protein, the XBB.1.5 protein, the BA.4/5 full-length S protein, the BQ.1.1 full-length S protein, the XBB.1.5 full-length S protein, the Mu-BA.5 protein, the Mu-BQ.1.1 protein, the Mu-XBB.1.5 protein, the full-length S protein after the BA.4/5 amino acid mutation, the full-length S protein after the BQ.1.1 amino acid mutation and the full-length S protein after the XBB.1.5 amino acid mutation are constructed based on at least one amino acid sequence as shown in SEQ ID No.31, SEQ ID No.33 and SEQ ID No.1-SEQ ID No.24.

The present invention further provides a vaccine for preventing and/or treating infection by a SARS-CoV-2 Omicron variant and a subtype thereof, comprising the protein, the precursor and/or the protein composition, as well as a pharmaceutically acceptable excipient or adjuvant component.

Further, the adjuvant component is an immunologic adjuvant.

Preferably, the immunologic adjuvant is selected from at least one of the following: squalene-based oil-in-water emulsion, aluminum salt, calcium salt, phytosaponin, phytopolysaccharide, monophosphate lipid A, muramyl dipeptide, muramyl tripeptide, bacterial toxin, GM-CSF cytokine, lipid and cationic liposomal material.

Further, the squalene-based oil-in-water emulsion is MF59; the aluminum salt is selected from at least one of aluminum hydroxide and alum; the calcium salt is tricalcium phosphate; the phytosaponin is either QS-21 or ISCOM; the phytopolysaccharide is astragalus polysaccharide; the bacterial toxin is selected from at least one of recombinant cholera toxin and diphtheria toxin; the lipid is selected from at least one of the following: phosphatidylethanolamine, phosphatidylcholine, cholesterol, dioleoylphosphatidylethanolamine; the cationic liposome material is selected from at least one of the following: (2,3-dioleoyloxypropyl) trimethyl ammonium chloride, N-[1-(2,3-dioleoyl chloride) propyl]-N,N,N-trimethylamine chloride, cationic cholesterol, dimethyl-2,3-dioleyloxypropyl-2-(2-spermidinecarboxamido)ethyl ammonium trifluoroacetate, trimethyldodecylammonium bromide, trimethyltetradecylammonium bromide, trimethylhexadecylammonium bromide, dimethyldioctadecylammonium bromide, and CpG ODN.

Further, the vaccine is an injection preparation, a nasal spray preparation and an oral preparation. Further, the vaccine is an intramuscular injection preparation.

The present invention further provides use of the protein, the precursor and/or the protein composition or the vaccine in preparing drugs for treating and/or preventing SARS-CoV-2 or mutant infection or pathogenicity thereof.

Further, the SARS-CoV-2 mutant is selected from any one of Alpha, Beta, Gamma, Delta or Omicron strain, and Omicron strain includes at least one of BA.1, BA.2, BA.2.12.1, BA.4, BA.5, BQ.1.1 and XBB.1.5.

The present invention further provides a mRNA vaccine for preventing and/or treating infection by a SARS-CoV-2 Omicron variant strain and a subtype thereof, comprising a polynucleotide sequence encoding any one amino acid of SEQ ID No.1-SEQ ID No.24.

The present invention further provides an adenovirus vaccine for preventing and/or treating infection by a SARS-CoV-2 Omicron variant strain and a subtype thereof, comprising a polynucleotide sequence encoding any one amino acid of SEQ ID No.1-SEQ ID No.24.

The following sequences are constructed by the applicant on the basis of SARS-CoV-2 or the mutant Delta thereof, the RBD sequence of the S protein in Omicron variant subline BA.4/5, BQ.1.1 and XBB.1.5 or constructed after mutating one or more sites.

Wherein, the recombinant protein or precursor formed on the basis of the RBD sequence of the S protein in Omicron variant subline BA.4/5, BQ.1.1 and XBB.1.5 and heptad repeat regions HR1 and HR2 of S protein of SARS-CoV-2 are defined as BA.5 protein, BQ.1.1 protein and XBB.1.5 protein, respectively.

The protein or precursor constructed on the basis of full-length S protein sequences of Omicron variant subline BA.4/5, BQ.1.1 and XBB.1.5 are defined as BA.4/5 full-length S protein, BQ.1.1 full-length S protein and XBB.1.5 full-length S protein, respectively.

The recombinant protein or precursor formed by introducing three amino acid mutations (F371S, P373S, F375S, with residue numbers counted from the full-length sequence of wild-type SARS-CoV-2) into the S protein RBD sequences of Omicron variant sublines BA.4/5, BQ.1.1, and XBB.1.5, followed by combination with the heptad repeat regions HR1 and HR2 of the SARS-CoV-2 S protein, is defined as the Mu-BA.5 protein, Mu-BQ.1.1 protein, and Mu-XBB.1.5 protein, respectively.

The full-length S protein or precursor constructed from the S protein RBD sequences of Omicron variant sublineages BA.4/5, BQ.1.1, and XBB.1.5, with three amino acid mutations (F371S, P373S, F375S), is defined as the BA.4/5 amino acid-mutated full-length S protein, the BQ.1.1 amino acid-mutated full-length S protein, and the XBB.1.5 amino acid-mutated full-length S protein, respectively.

Wherein, SEQ ID No.1-SEQ ID No.3 are RBD-HR1-HR2 amino acid sequences of Omicron variant subline BA.4/5, BQ.1.1 and XBB.1.5, respectively.

SEQ ID No.4-SEQ ID No.6 are S protein full-length amino acid sequences of Omicron variant subline BA.4/5, BQ.1.1 and XBB.1.5, respectively.

SEQ ID No.7-SEQ ID No.9 are RBD-HR1-HR2 amino acid sequences obtained when the RBD amino acid sequences of Omicron variant subline BA.4/5, BQ.1.1 and XBB.1.5 are subjected to the mutation of three amino acids (F371S, P373S, F375S), respectively.

SEQ ID No.10-SEQ ID No.12 are S protein full-length amino acid sequences obtained when the RBD amino acid sequences of Omicron variant subline BA.4/5, BQ.1.1 and XBB.1.5 are subjected to the mutation of three amino acids (F371S, P373S, F375S), respectively.

SEQ ID No.13-SEQ ID No.24 are RBD-HR1-HR2 amino acid sequences of Omicron variant subline BA.4/5, BQ.1.1 and XBB.1.5, the amino acid sequences adding the signal peptide-Trx tag-6His tag-EK enzyme before the mutated RBD-HR1-HR2 amino acid sequences, namely, SEQ ID No.13-SEQ ID No.15, SEQ ID No.19-SEQ ID No.21;
the signal peptide is added before the S protein full-length amino acid sequences of Omicron variant subline BA.4/5, BQ.1.1 and XBB.1.5 and mutated S protein full-length amino acid sequences, namely, SEQ ID No.16-SEQ ID No.18, SEQ ID No.22-SEQ ID No.24.

SEQ ID No.25-SEQ ID No.30 are nucleotide sequences of signal peptide-Trx tag-6His tag-EK enzyme amino acid sequences added before the RBD-HR1-HR2 amino acid sequences encoding the Omicron variant subline BA.4/5, BQ.1.1 and XBB.1.5, the mutated RBD-HR1-HR2 amino acid sequences (SEQ ID No.19-SEQ ID No.21), respectively.

SEQ ID No.31 is the precursor amino acid sequence of the Delta protein; SEQ ID No.32 is the nucleotide sequence encoding SEQ ID No.31; SEQ ID No.33 is the full-length amino acid sequence of Delta S antigen; SEQ ID No.34 is the full-length amino acid sequence of Omicron-BA.1 S antigen; SEQ ID No.35 is the full-length amino acid sequence of Omicron-BA.1 S antigen amino acid sequence mutation; SEQ ID No.36 is a Delta protein amino acid sequence; SEQ ID No.37 is the precursor amino acid sequence of the RBD_{BA.1} -HR protein; SEQ ID No.38 is the nucleotide sequence encoding SEQ ID No.37; and SEQ ID No.39 is the RBD_{BA.1} -HR protein amino acid sequence.

In the present invention, three amino acids (F371S, P373S, F375S) can be mutated on S protein RBD of Omicron variant subline BA.4/5, BQ.1.1 and XBB.1.5, so as to enhance the antiviral activity of the vaccine, and significantly increase the neutralizing antibody.
SEQ ID No.1: RBD-HR1-HR2 amino acid sequence of BA.5-Omicron variant subline BA.4/5 
VQPTESIVRFPNITNLCPFDEVFNATRFASVYAWNRKRISNCVADYSVLYNFAPFFAFKCY
GVSPTKLNDLCFTNVYADSFVIRGNEVSQIAPGQTGNIADYNYKLPDDFTGCVIAWNSN
KLDSKVGGNYNYRYRLFRKSNLKPFERDISTEIYQAGNKPCNGVAGVNCYFPLQSYGFR
PTYGVGHQPYRVVVLSFELLHAPATVCGPKKSTNLVKNKSVNFNFNGLYENQKLIANQF
NSAIGKIQDSLSSTASALGKLQDVVNQNAQALNTLVKQLKNHTSPDVDLGDISGINASV
VNIQKEIDRLNEVAKNLNESLIDLQEL
SEQ ID No.2: RBD-HR1-HR2 sequence of BQ.1.1-Omicron variant subline BQ.1.1 
VQPTESIVRFPNITNLCPFDEVFNATTFASVYAWNRKRISNCVADYSVLYNFAPFFAFKCY
GVSPTKLNDLCFTNVYADSFVIRGNEVSQIAPGQTGNIADYNYKLPDDFTGCVIAWNSN
KLDSTVGGNYNYRYRLFRKSKLKPFERDISTEIYQAGNKPCNGVAGVNCYFPLQSYGFR
PTYGVGHQPYRVVVLSFELLHAPATVCGPKKSTNLVKNKSVNFNFNGLYENQKLIANQF
NSAIGKIQDSLSSTASALGKLQDVVNQNAQALNTLVKQLKNHTSPDVDLGDISGINASV
 VNIQKEIDRLNEVAKNLNESLIDLQEL
SEQ ID No.3: RBD-HR1-HR2 sequence of XBB.1.5-Omicron variant subline XBB.1.5 
VQPTESIVRFPNITNLCPFHEVFNATTFASVYAWNRKRISNCVADYSVIYNFAPFFAFKCY
GVSPTKLNDLCFTNVYADSFVIRGNEVSQIAPGQTGNIADYNYKLPDDFTGCVIAWNSN
KLDSKPSGNYNYLYRLFRKSKLKPFERDISTEIYQAGNKPCNGVAGPNCYSPLQSYGFRP
TYGVGHQPYRVVVLSFELLHAPATVCGPKKSTNLVKNKSVNFNFNGLYENQKLIANQFN
SAIGKIQDSLSSTASALGKLQDVVNQNAQALNTLVKQLKNHTSPDVDLGDISGINASVV
NIQKEIDRLNEVAKNLNESLIDLQEL
SEQ ID No.4: S protein full-length amino acid sequence of Omicron variant subline BA.4/5 
VNLITRTQSYTNSFTRGVYYPDKVFRSSVLHSTQDLFLPFFSNVTWFHAISGTNGTKRFD
NPVLPFNDGVYFASTEKSNIIRGWIFGTTLDSKTQSLLIVNNATNVVIKVCEFQFCNDPFL
DVYYHKNNKSWMESEFRVYSSANNCTFEYVSQPFLMDLEGKQGNFKNLREFVFKNIDG
YFKIYSKHTPINLGRDLPQGFSALEPLVDLPIGINITRFQTLLALHRSYLTPGDSSSGWTAG
AAAYYVGYLQPRTFLLKYNENGTITDAVDCALDPLSETKCTLKSFTVEKGIYQTSNFRVQ
PTESIVRFPNITNLCPFDEVFNATRFASVYAWNRKRISNCVADYSVLYNFAPFFAFKCYGV
SPTKLNDLCFTNVYADSFVIRGNEVSQIAPGQTGNIADYNYKLPDDFTGCVIAWNSNKL
DSKVGGNYNYRYRLFRKSNLKPFERDISTEIYQAGNKPCNGVAGVNCYFPLQSYGFRPT
YGVGHQPYRVVVLSFELLHAPATVCGPKKSTNLVKNKCVNFNFNGLTGTGVLTESNKKF
LPFQQFGRDIADTTDAVRDPQTLEILDITPCSFGGVSVITPGTNTSNQVAVLYQGVNCTEV
PVAIHADQLTPTWRVYSTGSNVFQTRAGCLIGAEYVNNSYECDIPIGAGICASYQTQTKS
HRRARSVASQSIIAYTMSLGAENSVAYSNNSIAIPTNFTISVTTEILPVSMTKTSVDCTMYI
CGDSTECSNLLLQYGSFCTQLKRALTGIAVEQDKNTQEVFAQVKQIYKTPPIKYFGGFNF
SQILPDPSKPSKRSFIEDLLFNKVTLADAGFIKQYGDCLGDIAARDLICAQKFNGLTVLPP
LLTDEMIAQYTSALLAGTITSGWTFGAGAALQIPFAMQMAYRFNGIGVTQNVLYENQKL
IANQFNSAIGKIQDSLSSTASALGKLQDVVNHNAQALNTLVKQLSSKFGAISSVLNDILSR
LDKVEAEVQIDRLITGRLQSLQTYVTQQLIRAAEIRASANLAATKMSECVLGQSKRVDFC
GKGYHLMSFPQSAPHGVVFLHVTYVPAQEKNFTTAPAICHDGKAHFPREGVFVSNGTH
WFVTQRNFYEPQIITTDNTFVSGNCDVVIGIVNNTVYDPLQPELDSFKEELDKYFKNHTS
 
PDVDLGDISGINASVVNIQKEIDRLNEVAKNLNESLIDLQELGKYEQYIKWPWYIWLGFI
AGLIAIVMVTIMLCCMTSCCSCLKGCCSCGSCCKFDEDDSEPVLKGVKLHYT
SEQ ID No.5: S protein full-length amino acid sequence of Omicron variant subline BQ.1.1 
VNLITRTQSYTNSFTRGVYYPDKVFRSSVLHSTQDLFLPFFSNVTWFHAISGTNGTKRFD
NPVLPFNDGVYFASTEKSNIIRGWIFGTTLDSKTQSLLIVNNATNVVIKVCEFQFCNDPFL
DVYYHKNNKSWMESEFRVYSSANNCTFEYVSQPFLMDLEGKQGNFKNLREFVFKNIDG
YFKIYSKHTPINLGRDLPQGFSALEPLVDLPIGINITRFQTLLALHRSYLTPGDSSSGWTAG
AAAYYVGYLQPRTFLLKYNENGTITDAVDCALDPLSETKCTLKSFTVEKGIYQTSNFRVQ
PTESIVRFPNITNLCPFDEVFNATTFASVYAWNRKRISNCVADYSVLYNFAPFFAFKCYGV
SPTKLNDLCFTNVYADSFVIRGNEVSQIAPGQTGNIADYNYKLPDDFTGCVIAWNSNKL
DSTVGGNYNYRYRLFRKSKLKPFERDISTEIYQAGNKPCNGVAGVNCYFPLQSYGFRPT
YGVGHQPYRVVVLSFELLHAPATVCGPKKSTNLVKNKCVNFNFNGLTGTGVLTESNKKF
LPFQQFGRDIADTTDAVRDPQTLEILDITPCSFGGVSVITPGTNTSNQVAVLYQGVNCTEV
PVAIHADQLTPTWRVYSTGSNVFQTRAGCLIGAEYVNNSYECDIPIGAGICASYQTQTKS
HRRARSVASQSIIAYTMSLGAENSVAYSNNSIAIPTNFTISVTTEILPVSMTKTSVDCTMYI
CGDSTECSNLLLQYGSFCTQLKRALTGIAVEQDKNTQEVFAQVKQIYKTPPIKYFGGFNF
SQILPDPSKPSKRSFIEDLLFNKVTLADAGFIKQYGDCLGDIAARDLICAQKFNGLTVLPP
LLTDEMIAQYTSALLAGTITSGWTFGAGAALQIPFAMQMAYRFNGIGVTQNVLYENQKL
IANQFNSAIGKIQDSLSSTASALGKLQDVVNHNAQALNTLVKQLSSKFGAISSVLNDILSR
LDKVEAEVQIDRLITGRLQSLQTYVTQQLIRAAEIRASANLAATKMSECVLGQSKRVDFC
GKGYHLMSFPQSAPHGVVFLHVTYVPAQEKNFTTAPAICHDGKAHFPREGVFVSNGTH
WFVTQRNFYEPQIITTDNTFVSGNCDVVIGIVNNTVYDPLQPELDSFKEELDKYFKNHTS
PDVDLGDISGINASVVNIQKEIDRLNEVAKNLNESLIDLQELGKYEQYIKWPWYIWLGFI
AGLIAIVMVTIMLCCMTSCCSCLKGCCSCGSCCKFDEDDSEPVLKGVKLHYT
SEQ ID No.6: S protein full-length amino acid sequence of Omicron variant subline XBB.1.5 
VNLITRTQSYTNSFTRGVYYPDKVFRSSVLHSTQDLFLPFFSNVTWFHAIHVSGTNGTKR
FDNPALPFNDGVYFASTEKSNIIRGWIFGTTLDSKTQSLLIVNNATNVVIKVCEFQFCNDP
FLDVYQKNNKSWMESEFRVYSSANNCTFEYVSQPFLMDLEGKEGNFKNLREFVFKNID
 
GYFKIYSKHTPINLERDLPQGFSALEPLVDLPIGINITRFQTLLALHRSYLTPVDSSSGWTA
GAAAYYVGYLQPRTFLLKYNENGTITDAVDCALDPLSETKCTLKSFTVEKGIYQTSNFRV
QPTESIVRFPNITNLCPFHEVFNATTFASVYAWNRKRISNCVADYSVIYNFAPFFAFKCYG
VSPTKLNDLCFTNVYADSFVIRGNEVSQIAPGQTGNIADYNYKLPDDFTGCVIAWNSNK
LDSKPSGNYNYLYRLFRKSKLKPFERDISTEIYQAGNKPCNGVAGPNCYSPLQSYGFRPT
YGVGHQPYRVVVLSFELLHAPATVCGPKKSTNLVKNKCVNFNFNGLTGTGVLTESNKKF
LPFQQFGRDIADTTDAVRDPQTLEILDITPCSFGGVSVITPGTNTSNQVAVLYQGVNCTEV
PVAIHADQLTPTWRVYSTGSNVFQTRAGCLIGAEYVNNSYECDIPIGAGICASYQTQTKS
HRRARSVASQSIIAYTMSLGAENSVAYSNNSIAIPTNFTISVTTEILPVSMTKTSVDCTMYI
CGDSTECSNLLLQYGSFCTQLKRALTGIAVEQDKNTQEVFAQVKQIYKTPPIKYFGGFNF
SQILPDPSKPSKRSFIEDLLFNKVTLADAGFIKQYGDCLGDIAARDLICAQKFNGLTVLPP
LLTDEMIAQYTSALLAGTITSGWTFGAGAALQIPFAMQMAYRFNGIGVTQNVLYENQKL
IANQFNSAIGKIQDSLSSTASALGKLQDVVNHNAQALNTLVKQLSSKFGAISSVLNDILSR
LDKVEAEVQIDRLITGRLQSLQTYVTQQLIRAAEIRASANLAATKMSECVLGQSKRVDFC
GKGYHLMSFPQSAPHGVVFLHVTYVPAQEKNFTTAPAICHDGKAHFPREGVFVSNGTH
WFVTQRNFYEPQIITTDNTFVSGNCDVVIGIVNNTVYDPLQPELDSFKEELDKYFKNHTS
PDVDLGDISGINASVVNIQKEIDRLNEVAKNLNESLIDLQELGKYEQYIKWPWYIWLGFI
AGLIAIVMVTIMLCCMTSCCSCLKGCCSCGSCCKFDEDDSEPVLKGVKLHYT
SEQ ID No.7: Mu-BA.5 (sequence after RBD mutation in RBD-HR1-HR2 amino acid sequence of Omicron variant subline BA.4/5) 
VQPTESIVRFPNITNLCPFDEVFNATRFASVYAWNRKRISNCVADYSVLYNSASFSAFKCY
GVSPTKLNDLCFTNVYADSFVIRGNEVSQIAPGQTGNIADYNYKLPDDFTGCVIAWNSN
KLDSKVGGNYNYRYRLFRKSNLKPFERDISTEIYQAGNKPCNGVAGVNCYFPLQSYGFR
PTYGVGHQPYRVVVLSFELLHAPATVCGPKKSTNLVKNKSVNFNFNGLYENQKLIANQF
NSAIGKIQDSLSSTASALGKLQDVVNQNAQALNTLVKQLKNHTSPDVDLGDISGINASV
VNIQKEIDRLNEVAKNLNESLIDLQEL
SEQ ID No.8: Mu-BQ.1.1 (sequence after RBD mutation in RBD-HR1-HR2 amino acid sequence of Omicron variant subline BQ.1.1) 
VQPTESIVRFPNITNLCPFDEVFNATTFASVYAWNRKRISNCVADYSVLYNSASFSAFKCY
GVSPTKLNDLCFTNVYADSFVIRGNEVSQIAPGQTGNIADYNYKLPDDFTGCVIAWNSN
KLDSTVGGNYNYRYRLFRKSKLKPFERDISTEIYQAGNKPCNGVAGVNCYFPLQSYGFR
PTYGVGHQPYRVVVLSFELLHAPATVCGPKKSTNLVKNKSVNFNFNGLYENQKLIANQF
NSAIGKIQDSLSSTASALGKLQDVVNQNAQALNTLVKQLKNHTSPDVDLGDISGINASV
VNIQKEIDRLNEVAKNLNESLIDLQEL
SEQ ID No.9: Mu-XBB.1.5 (sequence after RBD mutation in RBD-HR1-HR2 amino acid sequence of Omicron variant subline XBB.1.5) 
VQPTESIVRFPNITNLCPFHEVFNATTFASVYAWNRKRISNCVADYSVIYNSASFSAFKCY
GVSPTKLNDLCFTNVYADSFVIRGNEVSQIAPGQTGNIADYNYKLPDDFTGCVIAWNSN
KLDSKPSGNYNYLYRLFRKSKLKPFERDISTEIYQAGNKPCNGVAGPNCYSPLQSYGFRP
TYGVGHQPYRVVVLSFELLHAPATVCGPKKSTNLVKNKSVNFNFNGLYENQKLIANQFN
SAIGKIQDSLSSTASALGKLQDVVNQNAQALNTLVKQLKNHTSPDVDLGDISGINASVV
NIQKEIDRLNEVAKNLNESLIDLQEL
SEQ ID No.10: sequence after mutation of S protein full-length amino acid sequence of Omicron variant subline BA.4/5 
VNLITRTQSYTNSFTRGVYYPDKVFRSSVLHSTQDLFLPFFSNVTWFHAISGTNGTKRFD
NPVLPFNDGVYFASTEKSNIIRGWIFGTTLDSKTQSLLIVNNATNVVIKVCEFQFCNDPFL
DVYYHKNNKSWMESEFRVYSSANNCTFEYVSQPFLMDLEGKQGNFKNLREFVFKNIDG
YFKIYSKHTPINLGRDLPQGFSALEPLVDLPIGINITRFQTLLALHRSYLTPGDSSSGWTAG
AAAYYVGYLQPRTFLLKYNENGTITDAVDCALDPLSETKCTLKSFTVEKGIYQTSNFRVQ
PTESIVRFPNITNLCPFDEVFNATRFASVYAWNRKRISNCVADYSVLYNSASFSAFKCYGV
SPTKLNDLCFTNVYADSFVIRGNEVSQIAPGQTGNIADYNYKLPDDFTGCVIAWNSNKL
DSKVGGNYNYRYRLFRKSNLKPFERDISTEIYQAGNKPCNGVAGVNCYFPLQSYGFRPT
YGVGHQPYRVVVLSFELLHAPATVCGPKKSTNLVKNKCVNFNFNGLTGTGVLTESNKKF
LPFQQFGRDIADTTDAVRDPQTLEILDITPCSFGGVSVITPGTNTSNQVAVLYQGVNCTEV
PVAIHADQLTPTWRVYSTGSNVFQTRAGCLIGAEYVNNSYECDIPIGAGICASYQTQTKS
HRRARSVASQSIIAYTMSLGAENSVAYSNNSIAIPTNFTISVTTEILPVSMTKTSVDCTMYI
CGDSTECSNLLLQYGSFCTQLKRALTGIAVEQDKNTQEVFAQVKQIYKTPPIKYFGGFNF
 
SQILPDPSKPSKRSFIEDLLFNKVTLADAGFIKQYGDCLGDIAARDLICAQKFNGLTVLPP
LLTDEMIAQYTSALLAGTITSGWTFGAGAALQIPFAMQMAYRFNGIGVTQNVLYENQKL
IANQFNSAIGKIQDSLSSTASALGKLQDVVNHNAQALNTLVKQLSSKFGAISSVLNDILSR
LDKVEAEVQIDRLITGRLQSLQTYVTQQLIRAAEIRASANLAATKMSECVLGQSKRVDFC
GKGYHLMSFPQSAPHGVVFLHVTYVPAQEKNFTTAPAICHDGKAHFPREGVFVSNGTH
WFVTQRNFYEPQIITTDNTFVSGNCDVVIGIVNNTVYDPLQPELDSFKEELDKYFKNHTS
PDVDLGDISGINASVVNIQKEIDRLNEVAKNLNESLIDLQELGKYEQYIKWPWYIWLGFI
AGLIAIVMVTIMLCCMTSCCSCLKGCCSCGSCCKFDEDDSEPVLKGVKLHYT
SEQ ID No.11: sequence after mutation of S protein full-length amino acid sequence of Omicron variant subline BQ.1.1 
VNLITRTQSYTNSFTRGVYYPDKVFRSSVLHSTQDLFLPFFSNVTWFHAISGTNGTKRFD
NPVLPFNDGVYFASTEKSNIIRGWIFGTTLDSKTQSLLIVNNATNVVIKVCEFQFCNDPFL
DVYYHKNNKSWMESEFRVYSSANNCTFEYVSQPFLMDLEGKQGNFKNLREFVFKNIDG
YFKIYSKHTPINLGRDLPQGFSALEPLVDLPIGINITRFQTLLALHRSYLTPGDSSSGWTAG
AAAYYVGYLQPRTFLLKYNENGTITDAVDCALDPLSETKCTLKSFTVEKGIYQTSNFRVQ
PTESIVRFPNITNLCPFDEVFNATTFASVYAWNRKRISNCVADYSVLYNSASFSAFKCYGV
SPTKLNDLCFTNVYADSFVIRGNEVSQIAPGQTGNIADYNYKLPDDFTGCVIAWNSNKL
DSTVGGNYNYRYRLFRKSKLKPFERDISTEIYQAGNKPCNGVAGVNCYFPLQSYGFRPT
YGVGHQPYRVVVLSFELLHAPATVCGPKKSTNLVKNKCVNFNFNGLTGTGVLTESNKKF
LPFQQFGRDIADTTDAVRDPQTLEILDITPCSFGGVSVITPGTNTSNQVAVLYQGVNCTEV
PVAIHADQLTPTWRVYSTGSNVFQTRAGCLIGAEYVNNSYECDIPIGAGICASYQTQTKS
HRRARSVASQSIIAYTMSLGAENSVAYSNNSIAIPTNFTISVTTEILPVSMTKTSVDCTMYI
CGDSTECSNLLLQYGSFCTQLKRALTGIAVEQDKNTQEVFAQVKQIYKTPPIKYFGGFNF
SQILPDPSKPSKRSFIEDLLFNKVTLADAGFIKQYGDCLGDIAARDLICAQKFNGLTVLPP
LLTDEMIAQYTSALLAGTITSGWTFGAGAALQIPFAMQMAYRFNGIGVTQNVLYENQKL
IANQFNSAIGKIQDSLSSTASALGKLQDVVNHNAQALNTLVKQLSSKFGAISSVLNDILSR
LDKVEAEVQIDRLITGRLQSLQTYVTQQLIRAAEIRASANLAATKMSECVLGQSKRVDFC
GKGYHLMSFPQSAPHGVVFLHVTYVPAQEKNFTTAPAICHDGKAHFPREGVFVSNGTH
WFVTQRNFYEPQIITTDNTFVSGNCDVVIGIVNNTVYDPLQPELDSFKEELDKYFKNHTS
 
PDVDLGDISGINASVVNIQKEIDRLNEVAKNLNESLIDLQELGKYEQYIKWPWYIWLGFI
AGLIAIVMVTIMLCCMTSCCSCLKGCCSCGSCCKFDEDDSEPVLKGVKLHYT
SEQ ID No.12: sequence after mutation of S protein full-length amino acid sequence of Omicron variant subline XBB.1.5 
VNLITRTQSYTNSFTRGVYYPDKVFRSSVLHSTQDLFLPFFSNVTWFHAIHVSGTNGTKR
FDNPALPFNDGVYFASTEKSNIIRGWIFGTTLDSKTQSLLIVNNATNVVIKVCEFQFCNDP
FLDVYQKNNKSWMESEFRVYSSANNCTFEYVSQPFLMDLEGKEGNFKNLREFVFKNID
GYFKIYSKHTPINLERDLPQGFSALEPLVDLPIGINITRFQTLLALHRSYLTPVDSSSGWTA
GAAAYYVGYLQPRTFLLKYNENGTITDAVDCALDPLSETKCTLKSFTVEKGIYQTSNFRV
QPTESIVRFPNITNLCPFHEVFNATTFASVYAWNRKRISNCVADYSVIYNSASFSAFKCYG
VSPTKLNDLCFTNVYADSFVIRGNEVSQIAPGQTGNIADYNYKLPDDFTGCVIAWNSNK
LDSKPSGNYNYLYRLFRKSKLKPFERDISTEIYQAGNKPCNGVAGPNCYSPLQSYGFRPT
YGVGHQPYRVVVLSFELLHAPATVCGPKKSTNLVKNKCVNFNFNGLTGTGVLTESNKKF
LPFQQFGRDIADTTDAVRDPQTLEILDITPCSFGGVSVITPGTNTSNQVAVLYQGVNCTEV
PVAIHADQLTPTWRVYSTGSNVFQTRAGCLIGAEYVNNSYECDIPIGAGICASYQTQTKS
HRRARSVASQSIIAYTMSLGAENSVAYSNNSIAIPTNFTISVTTEILPVSMTKTSVDCTMYI
CGDSTECSNLLLQYGSFCTQLKRALTGIAVEQDKNTQEVFAQVKQIYKTPPIKYFGGFNF
SQILPDPSKPSKRSFIEDLLFNKVTLADAGFIKQYGDCLGDIAARDLICAQKFNGLTVLPP
LLTDEMIAQYTSALLAGTITSGWTFGAGAALQIPFAMQMAYRFNGIGVTQNVLYENQKL
IANQFNSAIGKIQDSLSSTASALGKLQDVVNHNAQALNTLVKQLSSKFGAISSVLNDILSR
LDKVEAEVQIDRLITGRLQSLQTYVTQQLIRAAEIRASANLAATKMSECVLGQSKRVDFC
GKGYHLMSFPQSAPHGVVFLHVTYVPAQEKNFTTAPAICHDGKAHFPREGVFVSNGTH
WFVTQRNFYEPQIITTDNTFVSGNCDVVIGIVNNTVYDPLQPELDSFKEELDKYFKNHTS
PDVDLGDISGINASVVNIQKEIDRLNEVAKNLNESLIDLQELGKYEQYIKWPWYIWLGFI
AGLIAIVMVTIMLCCMTSCCSCLKGCCSCGSCCKFDEDDSEPVLKGVKLHYT
SEQ ID No.13: signal peptide-Trx-6His-EK-RBD-HR1-HR2 amino acid sequence of BA.5 
MLLVNQSHQGFNKEHTSKMVSAIVLYVLLAAAAHSAFAADSIHIKDSDDLKNRLAEAG
DKLVVIDFMATWCGPCKMIGPKLDEMANEMSDSIVVLKVDVDECEDIATEYNINSMPTF
 
VFVKNSKKIEEFSGANVDKLRNTIIKLKLAGSGSGHMHHHHHHSSGDDDDKVQPTESIV
RFPNITNLCPFDEVFNATRFASVYAWNRKRISNCVADYSVLYNFAPFFAFKCYGVSPTKLN
DLCFTNVYADSFVIRGNEVSQIAPGQTGNIADYNYKLPDDFTGCVIAWNSNKLDSKVGG
NYNYRYRLFRKSNLKPFERDISTEIYQAGNKPCNGVAGVNCYFPLQSYGFRPTYGVGHQ
PYRVVVLSFELLHAPATVCGPKKSTNLVKNKSVNFNFNGLYENQKLIANQFNSAIGKIQD
SLSSTASALGKLQDVVNQNAQALNTLVKQLKNHTSPDVDLGDISGINASVVNIQKEIDRL
NEVAKNLNESLIDLQEL
SEQ ID No.14: signal peptide-Trx-6His-EK-RBD-HR1-HR2 amino acid sequence of BQ.1.1 
MLLVNQSHQGFNKEHTSKMVSAIVLYVLLAAAAHSAFAADSIHIKDSDDLKNRLAEAG
DKLVVIDFMATWCGPCKMIGPKLDEMANEMSDSIVVLKVDVDECEDIATEYNINSMPTF
VFVKNSKKIEEFSGANVDKLRNTIIKLKLAGSGSGHMHHHHHHSSGDDDDKVQPTESIV
RFPNITNLCPFDEVFNATTFASVYAWNRKRISNCVADYSVLYNFAPFFAFKCYGVSPTKLN
DLCFTNVYADSFVIRGNEVSQIAPGQTGNIADYNYKLPDDFTGCVIAWNSNKLDSTVGG
NYNYRYRLFRKSKLKPFERDISTEIYQAGNKPCNGVAGVNCYFPLQSYGFRPTYGVGHQ
PYRVVVLSFELLHAPATVCGPKKSTNLVKNKSVNFNFNGLYENQKLIANQFNSAIGKIQD
SLSSTASALGKLQDVVNQNAQALNTLVKQLKNHTSPDVDLGDISGINASVVNIQKEIDRL
NEVAKNLNESLIDLQEL
SEQ ID No.15: signal peptide-Trx-6His-EK-RBD-HR1-HR2 amino acid sequence of XBB.1.5 
MLLVNQSHQGFNKEHTSKMVSAIVLYVLLAAAAHSAFAADSIHIKDSDDLKNRLAEAG
DKLVVIDFMATWCGPCKMIGPKLDEMANEMSDSIVVLKVDVDECEDIATEYNINSMPTF
VFVKNSKKIEEFSGANVDKLRNTIIKLKLAGSGSGHMHHHHHHSSGDDDDKVQPTESIV
RFPNITNLCPFHEVFNATTFASVYAWNRKRISNCVADYSVIYNFAPFFAFKCYGVSPTKLN
DLCFTNVYADSFVIRGNEVSQIAPGQTGNIADYNYKLPDDFTGCVIAWNSNKLDSKPSG
NYNYLYRLFRKSKLKPFERDISTEIYQAGNKPCNGVAGPNCYSPLQSYGFRPTYGVGHQP
YRVVVLSFELLHAPATVCGPKKSTNLVKNKSVNFNFNGLYENQKLIANQFNSAIGKIQDS
LSSTASALGKLQDVVNQNAQALNTLVKQLKNHTSPDVDLGDISGINASVVNIQKEIDRL
NEVAKNLNESLIDLQEL
SEQ ID No.16: amino acid sequence adding signal peptide before S protein full-length amino acid sequence of Omicron variant subline BA.4/5 
MFVFLVLLPLVSSQCVNLITRTQSYTNSFTRGVYYPDKVFRSSVLHSTQDLFLPFFSNVT
WFHAISGTNGTKRFDNPVLPFNDGVYFASTEKSNIIRGWIFGTTLDSKTQSLLIVNNATNV
VIKVCEFQFCNDPFLDVYYHKNNKSWMESEFRVYSSANNCTFEYVSQPFLMDLEGKQG
NFKNLREFVFKNIDGYFKIYSKHTPINLGRDLPQGFSALEPLVDLPIGINITRFQTLLALHR
SYLTPGDSSSGWTAGAAAYYVGYLQPRTFLLKYNENGTITDAVDCALDPLSETKCTLKSF
TVEKGIYQTSNFRVQPTESIVRFPNITNLCPFDEVFNATRFASVYAWNRKRISNCVADYSV
LYNFAPFFAFKCYGVSPTKLNDLCFTNVYADSFVIRGNEVSQIAPGQTGNIADYNYKLPD
DFTGCVIAWNSNKLDSKVGGNYNYRYRLFRKSNLKPFERDISTEIYQAGNKPCNGVAGV
NCYFPLQSYGFRPTYGVGHQPYRVVVLSFELLHAPATVCGPKKSTNLVKNKCVNFNFNG
LTGTGVLTESNKKFLPFQQFGRDIADTTDAVRDPQTLEILDITPCSFGGVSVITPGTNTSNQ
VAVLYQGVNCTEVPVAIHADQLTPTWRVYSTGSNVFQTRAGCLIGAEYVNNSYECDIPIG
AGICASYQTQTKSHRRARSVASQSIIAYTMSLGAENSVAYSNNSIAIPTNFTISVTTEILPVS
MTKTSVDCTMYICGDSTECSNLLLQYGSFCTQLKRALTGIAVEQDKNTQEVFAQVKQIY
KTPPIKYFGGFNFSQILPDPSKPSKRSFIEDLLFNKVTLADAGFIKQYGDCLGDIAARDLIC
AQKFNGLTVLPPLLTDEMIAQYTSALLAGTITSGWTFGAGAALQIPFAMQMAYRFNGIG
VTQNVLYENQKLIANQFNSAIGKIQDSLSSTASALGKLQDVVNHNAQALNTLVKQLSSK
FGAISSVLNDILSRLDKVEAEVQIDRLITGRLQSLQTYVTQQLIRAAEIRASANLAATKMS
ECVLGQSKRVDFCGKGYHLMSFPQSAPHGVVFLHVTYVPAQEKNFTTAPAICHDGKAHF
PREGVFVSNGTHWFVTQRNFYEPQIITTDNTFVSGNCDVVIGIVNNTVYDPLQPELDSFK
EELDKYFKNHTSPDVDLGDISGINASVVNIQKEIDRLNEVAKNLNESLIDLQELGKYEQYI
KWPWYIWLGFIAGLIAIVMVTIMLCCMTSCCSCLKGCCSCGSCCKFDEDDSEPVLKGVK
LHYT
SEQ ID No.17: amino acid sequence adding signal peptide after S protein full-length amino acid sequence of Omicron variant subline BQ.1.1 
MFVFLVLLPLVSSQCVNLITRTQSYTNSFTRGVYYPDKVFRSSVLHSTQDLFLPFFSNVT
WFHAISGTNGTKRFDNPVLPFNDGVYFASTEKSNIIRGWIFGTTLDSKTQSLLIVNNATNV
VIKVCEFQFCNDPFLDVYYHKNNKSWMESEFRVYSSANNCTFEYVSQPFLMDLEGKQG
 
NFKNLREFVFKNIDGYFKIYSKHTPINLGRDLPQGFSALEPLVDLPIGINITRFQTLLALHR
SYLTPGDSSSGWTAGAAAYYVGYLQPRTFLLKYNENGTITDAVDCALDPLSETKCTLKSF
TVEKGIYQTSNFRVQPTESIVRFPNITNLCPFDEVFNATTFASVYAWNRKRISNCVADYSV
LYNFAPFFAFKCYGVSPTKLNDLCFTNVYADSFVIRGNEVSQIAPGQTGNIADYNYKLPD
DFTGCVIAWNSNKLDSTVGGNYNYRYRLFRKSKLKPFERDISTEIYQAGNKPCNGVAGV
NCYFPLQSYGFRPTYGVGHQPYRVVVLSFELLHAPATVCGPKKSTNLVKNKCVNFNFNG
LTGTGVLTESNKKFLPFQQFGRDIADTTDAVRDPQTLEILDITPCSFGGVSVITPGTNTSNQ
VAVLYQGVNCTEVPVAIHADQLTPTWRVYSTGSNVFQTRAGCLIGAEYVNNSYECDIPIG
AGICASYQTQTKSHRRARSVASQSIIAYTMSLGAENSVAYSNNSIAIPTNFTISVTTEILPVS
MTKTSVDCTMYICGDSTECSNLLLQYGSFCTQLKRALTGIAVEQDKNTQEVFAQVKQIY
KTPPIKYFGGFNFSQILPDPSKPSKRSFIEDLLFNKVTLADAGFIKQYGDCLGDIAARDLIC
AQKFNGLTVLPPLLTDEMIAQYTSALLAGTITSGWTFGAGAALQIPFAMQMAYRFNGIG
VTQNVLYENQKLIANQFNSAIGKIQDSLSSTASALGKLQDVVNHNAQALNTLVKQLSSK
FGAISSVLNDILSRLDKVEAEVQIDRLITGRLQSLQTYVTQQLIRAAEIRASANLAATKMS
ECVLGQSKRVDFCGKGYHLMSFPQSAPHGVVFLHVTYVPAQEKNFTTAPAICHDGKAHF
PREGVFVSNGTHWFVTQRNFYEPQIITTDNTFVSGNCDVVIGIVNNTVYDPLQPELDSFK
EELDKYFKNHTSPDVDLGDISGINASVVNIQKEIDRLNEVAKNLNESLIDLQELGKYEQYI
KWPWYIWLGFIAGLIAIVMVTIMLCCMTSCCSCLKGCCSCGSCCKFDEDDSEPVLKGVK
LHYT
SEQ ID No.18: amino acid sequence adding signal peptide after S protein full-length amino acid sequence of Omicron variant subline XBB.1.5 
MFVFLVLLPLVSSQCVNLITRTQSYTNSFTRGVYYPDKVFRSSVLHSTQDLFLPFFSNVT
WFHAIHVSGTNGTKRFDNPALPFNDGVYFASTEKSNIIRGWIFGTTLDSKTQSLLIVNNAT
NVVIKVCEFQFCNDPFLDVYQKNNKSWMESEFRVYSSANNCTFEYVSQPFLMDLEGKE
GNFKNLREFVFKNIDGYFKIYSKHTPINLERDLPQGFSALEPLVDLPIGINITRFQTLLALH
RSYLTPVDSSSGWTAGAAAYYVGYLQPRTFLLKYNENGTITDAVDCALDPLSETKCTLK
SFTVEKGIYQTSNFRVQPTESIVRFPNITNLCPFHEVFNATTFASVYAWNRKRISNCVADYS
VIYNFAPFFAFKCYGVSPTKLNDLCFTNVYADSFVIRGNEVSQIAPGQTGNIADYNYKLP
DDFTGCVIAWNSNKLDSKPSGNYNYLYRLFRKSKLKPFERDISTEIYQAGNKPCNGVAGP
 
NCYSPLQSYGFRPTYGVGHQPYRVVVLSFELLHAPATVCGPKKSTNLVKNKCVNFNFNG
LTGTGVLTESNKKFLPFQQFGRDIADTTDAVRDPQTLEILDITPCSFGGVSVITPGTNTSNQ
VAVLYQGVNCTEVPVAIHADQLTPTWRVYSTGSNVFQTRAGCLIGAEYVNNSYECDIPIG
AGICASYQTQTKSHRRARSVASQSIIAYTMSLGAENSVAYSNNSIAIPTNFTISVTTEILPVS
MTKTSVDCTMYICGDSTECSNLLLQYGSFCTQLKRALTGIAVEQDKNTQEVFAQVKQIY
KTPPIKYFGGFNFSQILPDPSKPSKRSFIEDLLFNKVTLADAGFIKQYGDCLGDIAARDLIC
AQKFNGLTVLPPLLTDEMIAQYTSALLAGTITSGWTFGAGAALQIPFAMQMAYRFNGIG
VTQNVLYENQKLIANQFNSAIGKIQDSLSSTASALGKLQDVVNHNAQALNTLVKQLSSK
FGAISSVLNDILSRLDKVEAEVQIDRLITGRLQSLQTYVTQQLIRAAEIRASANLAATKMS
ECVLGQSKRVDFCGKGYHLMSFPQSAPHGVVFLHVTYVPAQEKNFTTAPAICHDGKAHF
PREGVFVSNGTHWFVTQRNFYEPQIITTDNTFVSGNCDVVIGIVNNTVYDPLQPELDSFK
EELDKYFKNHTSPDVDLGDISGINASVVNIQKEIDRLNEVAKNLNESLIDLQELGKYEQYI
KWPWYIWLGFIAGLIAIVMVTIMLCCMTSCCSCLKGCCSCGSCCKFDEDDSEPVLKGVK
LHYT
SEQ ID No.19: signal peptide Trx-6His-EK-RBD-HR1-HR2 amino acid sequence of Mu-BA.5 
MLLVNQSHQGFNKEHTSKMVSAIVLYVLLAAAAHSAFAADSIHIKDSDDLKNRLAEAG
DKLVVIDFMATWCGPCKMIGPKLDEMANEMSDSIVVLKVDVDECEDIATEYNINSMPTF
VFVKNSKKIEEFSGANVDKLRNTIIKLKLAGSGSGHMHHHHHHSSGDDDDKVQPTESIV
RFPNITNLCPFDEVFNATRFASVYAWNRKRISNCVADYSVLYNSASFSAFKCYGVSPTKL
NDLCFTNVYADSFVIRGNEVSQIAPGQTGNIADYNYKLPDDFTGCVIAWNSNKLDSKVG
GNYNYRYRLFRKSNLKPFERDISTEIYQAGNKPCNGVAGVNCYFPLQSYGFRPTYGVGH
QPYRVVVLSFELLHAPATVCGPKKSTNLVKNKSVNFNFNGLYENQKLIANQFNSAIGKIQ
DSLSSTASALGKLQDVVNQNAQALNTLVKQLKNHTSPDVDLGDISGINASVVNIQKEID
RLNEVAKNLNESLIDLQEL
SEQ ID No.20: signal peptide Trx-6His-EK-RBD-HR1-HR2 amino acid sequence of Mu-BQ.1.1 
MLLVNQSHQGFNKEHTSKMVSAIVLYVLLAAAAHSAFAADSIHIKDSDDLKNRLAEAG
DKLVVIDFMATWCGPCKMIGPKLDEMANEMSDSIVVLKVDVDECEDIATEYNINSMPTF
VFVKNSKKIEEFSGANVDKLRNTIIKLKLAGSGSGHMHHHHHHSSGDDDDKVQPTESIV
 
RFPNITNLCPFDEVFNATTFASVYAWNRKRISNCVADYSVLYNSASFSAFKCYGVSPTKL
NDLCFTNVYADSFVIRGNEVSQIAPGQTGNIADYNYKLPDDFTGCVIAWNSNKLDSTVG
GNYNYRYRLFRKSKLKPFERDISTEIYQAGNKPCNGVAGVNCYFPLQSYGFRPTYGVGH
QPYRVVVLSFELLHAPATVCGPKKSTNLVKNKSVNFNFNGLYENQKLIANQFNSAIGKIQ
DSLSSTASALGKLQDVVNQNAQALNTLVKQLKNHTSPDVDLGDISGINASVVNIQKEID
RLNEVAKNLNESLIDLQEL
SEQ ID No.21: signal peptide Trx-6His-EK-RBD-HR1-HR2 amino acid sequence of Mu-XBB.1.5 
MLLVNQSHQGFNKEHTSKMVSAIVLYVLLAAAAHSAFAADSIHIKDSDDLKNRLAEAG
DKLVVIDFMATWCGPCKMIGPKLDEMANEMSDSIVVLKVDVDECEDIATEYNINSMPTF
VFVKNSKKIEEFSGANVDKLRNTIIKLKLAGSGSGHMHHHHHHSSGDDDDKVQPTESIV
RFPNITNLCPFHEVFNATTFASVYAWNRKRISNCVADYSVIYNSASFSAFKCYGVSPTKLN
DLCFTNVYADSFVIRGNEVSQIAPGQTGNIADYNYKLPDDFTGCVIAWNSNKLDSKPSG
NYNYLYRLFRKSKLKPFERDISTEIYQAGNKPCNGVAGPNCYSPLQSYGFRPTYGVGHQP
YRVVVLSFELLHAPATVCGPKKSTNLVKNKSVNFNFNGLYENQKLIANQFNSAIGKIQDS
LSSTASALGKLQDVVNQNAQALNTLVKQLKNHTSPDVDLGDISGINASVVNIQKEIDRL
NEVAKNLNESLIDLQEL
SEQ ID No.22: amino acid sequence adding signal peptide before S protein full-length amino acid sequence of mutated Omicron variant subline BA.4/5 
MFVFLVLLPLVSSQCVNLITRTQSYTNSFTRGVYYPDKVFRSSVLHSTQDLFLPFFSNVT
WFHAISGTNGTKRFDNPVLPFNDGVYFASTEKSNIIRGWIFGTTLDSKTQSLLIVNNATNV
VIKVCEFQFCNDPFLDVYYHKNNKSWMESEFRVYSSANNCTFEYVSQPFLMDLEGKQG
NFKNLREFVFKNIDGYFKIYSKHTPINLGRDLPQGFSALEPLVDLPIGINITRFQTLLALHR
SYLTPGDSSSGWTAGAAAYYVGYLQPRTFLLKYNENGTITDAVDCALDPLSETKCTLKSF
TVEKGIYQTSNFRVQPTESIVRFPNITNLCPFDEVFNATRFASVYAWNRKRISNCVADYSV
LYNSASFSAFKCYGVSPTKLNDLCFTNVYADSFVIRGNEVSQIAPGQTGNIADYNYKLPD
DFTGCVIAWNSNKLDSKVGGNYNYRYRLFRKSNLKPFERDISTEIYQAGNKPCNGVAGV
NCYFPLQSYGFRPTYGVGHQPYRVVVLSFELLHAPATVCGPKKSTNLVKNKCVNFNFNG
 
LTGTGVLTESNKKFLPFQQFGRDIADTTDAVRDPQTLEILDITPCSFGGVSVITPGTNTSNQ
VAVLYQGVNCTEVPVAIHADQLTPTWRVYSTGSNVFQTRAGCLIGAEYVNNSYECDIPIG
AGICASYQTQTKSHRRARSVASQSIIAYTMSLGAENSVAYSNNSIAIPTNFTISVTTEILPVS
MTKTSVDCTMYICGDSTECSNLLLQYGSFCTQLKRALTGIAVEQDKNTQEVFAQVKQIY
KTPPIKYFGGFNFSQILPDPSKPSKRSFIEDLLFNKVTLADAGFIKQYGDCLGDIAARDLIC
AQKFNGLTVLPPLLTDEMIAQYTSALLAGTITSGWTFGAGAALQIPFAMQMAYRFNGIG
VTQNVLYENQKLIANQFNSAIGKIQDSLSSTASALGKLQDVVNHNAQALNTLVKQLSSK
FGAISSVLNDILSRLDKVEAEVQIDRLITGRLQSLQTYVTQQLIRAAEIRASANLAATKMS
ECVLGQSKRVDFCGKGYHLMSFPQSAPHGVVFLHVTYVPAQEKNFTTAPAICHDGKAHF
PREGVFVSNGTHWFVTQRNFYEPQIITTDNTFVSGNCDVVIGIVNNTVYDPLQPELDSFK
EELDKYFKNHTSPDVDLGDISGINASVVNIQKEIDRLNEVAKNLNESLIDLQELGKYEQYI
KWPWYIWLGFIAGLIAIVMVTIMLCCMTSCCSCLKGCCSCGSCCKFDEDDSEPVLKGVK
LHYT
SEQ ID No.23: amino acid sequence adding signal peptide before S protein full-length amino acid sequence of mutated Omicron variant subline BQ.1.1 
MFVFLVLLPLVSSQCVNLITRTQSYTNSFTRGVYYPDKVFRSSVLHSTQDLFLPFFSNVT
WFHAISGTNGTKRFDNPVLPFNDGVYFASTEKSNIIRGWIFGTTLDSKTQSLLIVNNATNV
VIKVCEFQFCNDPFLDVYYHKNNKSWMESEFRVYSSANNCTFEYVSQPFLMDLEGKQG
NFKNLREFVFKNIDGYFKIYSKHTPINLGRDLPQGFSALEPLVDLPIGINITRFQTLLALHR
SYLTPGDSSSGWTAGAAAYYVGYLQPRTFLLKYNENGTITDAVDCALDPLSETKCTLKSF
TVEKGIYQTSNFRVQPTESIVRFPNITNLCPFDEVFNATTFASVYAWNRKRISNCVADYSV
LYNSASFSAFKCYGVSPTKLNDLCFTNVYADSFVIRGNEVSQIAPGQTGNIADYNYKLPD
DFTGCVIAWNSNKLDSTVGGNYNYRYRLFRKSKLKPFERDISTEIYQAGNKPCNGVAGV
NCYFPLQSYGFRPTYGVGHQPYRVVVLSFELLHAPATVCGPKKSTNLVKNKCVNFNFNG
LTGTGVLTESNKKFLPFQQFGRDIADTTDAVRDPQTLEILDITPCSFGGVSVITPGTNTSNQ
VAVLYQGVNCTEVPVAIHADQLTPTWRVYSTGSNVFQTRAGCLIGAEYVNNSYECDIPIG
AGICASYQTQTKSHRRARSVASQSIIAYTMSLGAENSVAYSNNSIAIPTNFTISVTTEILPVS
MTKTSVDCTMYICGDSTECSNLLLQYGSFCTQLKRALTGIAVEQDKNTQEVFAQVKQIY
KTPPIKYFGGFNFSQILPDPSKPSKRSFIEDLLFNKVTLADAGFIKQYGDCLGDIAARDLIC
 
AQKFNGLTVLPPLLTDEMIAQYTSALLAGTITSGWTFGAGAALQIPFAMQMAYRFNGIG
VTQNVLYENQKLIANQFNSAIGKIQDSLSSTASALGKLQDVVNHNAQALNTLVKQLSSK
FGAISSVLNDILSRLDKVEAEVQIDRLITGRLQSLQTYVTQQLIRAAEIRASANLAATKMS
ECVLGQSKRVDFCGKGYHLMSFPQSAPHGVVFLHVTYVPAQEKNFTTAPAICHDGKAHF
PREGVFVSNGTHWFVTQRNFYEPQIITTDNTFVSGNCDVVIGIVNNTVYDPLQPELDSFK
EELDKYFKNHTSPDVDLGDISGINASVVNIQKEIDRLNEVAKNLNESLIDLQELGKYEQYI
KWPWYIWLGFIAGLIAIVMVTIMLCCMTSCCSCLKGCCSCGSCCKFDEDDSEPVLKGVK
LHYT
SEQ ID No.24: amino acid sequence adding signal peptide before S protein full-length amino acid sequence of mutated Omicron variant subline XBB.1.5 
MFVFLVLLPLVSSQCVNLITRTQSYTNSFTRGVYYPDKVFRSSVLHSTQDLFLPFFSNVT
WFHAIHVSGTNGTKRFDNPALPFNDGVYFASTEKSNIIRGWIFGTTLDSKTQSLLIVNNAT
NVVIKVCEFQFCNDPFLDVYQKNNKSWMESEFRVYSSANNCTFEYVSQPFLMDLEGKE
GNFKNLREFVFKNIDGYFKIYSKHTPINLERDLPQGFSALEPLVDLPIGINITRFQTLLALH
RSYLTPVDSSSGWTAGAAAYYVGYLQPRTFLLKYNENGTITDAVDCALDPLSETKCTLK
SFTVEKGIYQTSNFRVQPTESIVRFPNITNLCPFHEVFNATTFASVYAWNRKRISNCVADYS
VIYNSASFSAFKCYGVSPTKLNDLCFTNVYADSFVIRGNEVSQIAPGQTGNIADYNYKLP
DDFTGCVIAWNSNKLDSKPSGNYNYLYRLFRKSKLKPFERDISTEIYQAGNKPCNGVAGP
NCYSPLQSYGFRPTYGVGHQPYRVVVLSFELLHAPATVCGPKKSTNLVKNKCVNFNFNG
LTGTGVLTESNKKFLPFQQFGRDIADTTDAVRDPQTLEILDITPCSFGGVSVITPGTNTSNQ
VAVLYQGVNCTEVPVAIHADQLTPTWRVYSTGSNVFQTRAGCLIGAEYVNNSYECDIPIG
AGICASYQTQTKSHRRARSVASQSIIAYTMSLGAENSVAYSNNSIAIPTNFTISVTTEILPVS
MTKTSVDCTMYICGDSTECSNLLLQYGSFCTQLKRALTGIAVEQDKNTQEVFAQVKQIY
KTPPIKYFGGFNFSQILPDPSKPSKRSFIEDLLFNKVTLADAGFIKQYGDCLGDIAARDLIC
AQKFNGLTVLPPLLTDEMIAQYTSALLAGTITSGWTFGAGAALQIPFAMQMAYRFNGIG
VTQNVLYENQKLIANQFNSAIGKIQDSLSSTASALGKLQDVVNHNAQALNTLVKQLSSK
FGAISSVLNDILSRLDKVEAEVQIDRLITGRLQSLQTYVTQQLIRAAEIRASANLAATKMS
ECVLGQSKRVDFCGKGYHLMSFPQSAPHGVVFLHVTYVPAQEKNFTTAPAICHDGKAHF
PREGVFVSNGTHWFVTQRNFYEPQIITTDNTFVSGNCDVVIGIVNNTVYDPLQPELDSFK
 
EELDKYFKNHTSPDVDLGDISGINASVVNIQKEIDRLNEVAKNLNESLIDLQELGKYEQYI
KWPWYIWLGFIAGLIAIVMVTIMLCCMTSCCSCLKGCCSCGSCCKFDEDDSEPVLKGVK
LHYT
SEQ ID No.25: nucleotide sequence encoding SEQ ID No.13 (construct BA.4/5 protein) 
ATGCTGCTGGTGAACCAGAGCCACCAGGGTTTCAACAAGGAACACACTAGCAAGAT
GGTGTCTGCTATCGTCCTCTACGTGCTGCTCGCCGCCGCTGCTCACTCAGCTTTCGCC
GCTGACTCTATCCACATCAAGGACTCCGACGACCTCAAGAACCGTTTGGCCGAAGCC
GGCGACAAGCTGGTCGTTATCGACTTCATGGCTACCTGGTGTGGCCCTTGCAAGATGA
TCGGACCTAAGCTGGACGAAATGGCTAACGAGATGAGCGACAGCATCGTCGTTCTGA
AGGTGGACGTCGACGAATGTGAGGACATCGCCACTGAGTACAACATCAACAGCATGC
CTACCTTCGTTTTCGTGAAGAACTCTAAGAAGATCGAGGAGTTCTCTGGTGCCAACG
TGGACAAGCTCCGTAACACCATCATCAAGCTCAAGCTGGCCGGCAGCGGTAGCGGTC
ACATGCACCACCACCACCACCATTCAAGCGGCGACGACGACGACAAGGTGCAGCCA
ACCGAATCAATCGTCCGCTTCCCAAACATCACTAACCTGTGCCCATTCGACGAGGTGT
TCAACGCCACCCGTTTCGCCTCTGTCTACGCTTGGAACCGCAAGCGCATCTCCAACT
GTGTCGCCGACTACAGCGTGCTGTACAACTTCGCTCCTTTCTTCGCATTCAAGTGTTA
CGGAGTGTCCCCTACCAAGCTCAACGATTTGTGCTTCACAAACGTGTACGCTGACAG
CTTCGTGATCCGTGGTAACGAGGTTTCTCAAATCGCCCCTGGTCAGACAGGTAACATC
GCTGACTACAACTACAAGCTCCCAGACGACTTCACTGGCTGTGTTATCGCTTGGAACT
CAAACAAGCTGGACTCTAAGGTGGGTGGTAACTACAACTACCGGTACCGTCTGTTCC
GCAAGAGCAACCTCAAGCCATTCGAACGTGACATCAGCACAGAGATCTACCAAGCC
GGTAACAAGCCCTGCAACGGCGTTGCCGGAGTCAACTGCTACTTCCCCCTCCAGTCC
TACGGATTCCGCCCAACCTACGGCGTGGGTCACCAGCCATACCGCGTGGTTGTGCTG
AGCTTCGAACTGCTGCACGCCCCCGCTACCGTGTGTGGCCCTAAGAAGAGCACTAAC
CTCGTTAAGAACAAGTCCGTGAACTTCAACTTCAACGGTCTCTACGAAAACCAGAAG
CTGATCGCTAACCAGTTCAACTCCGCCATCGGCAAGATCCAGGACTCCCTGTCCTCCA
CAGCTTCCGCTCTGGGCAAGCTGCAGGACGTTGTGAACCAAAACGCTCAGGCCCTG
AACACACTGGTGAAGCAACTGAAGAACCACACCAGCCCCGACGTTGACCTGGGTGA
CATCAGCGGTATCAACGCTAGCGTGGTGAACATCCAGAAGGAGATCGACAGGCTGAA
 CGAAGTGGCTAAGAACTTGAACGAGAGCCTCATCGACCTGCAGGAACTGTAA
SEQ ID No.26: nucleotide sequence encoding SEQ ID No.14 (construct BQ.1.1 protein) 
ATGCTGCTGGTGAACCAGAGCCACCAGGGTTTCAACAAGGAACACACTAGCAAGAT
GGTGTCTGCTATCGTCCTCTACGTGCTGCTCGCCGCCGCTGCTCACTCAGCTTTCGCC
GCTGACTCTATCCACATCAAGGACTCCGACGACCTCAAGAACCGTTTGGCCGAAGCC
GGCGACAAGCTGGTCGTTATCGACTTCATGGCTACCTGGTGTGGCCCTTGCAAGATGA
TCGGACCTAAGCTGGACGAAATGGCTAACGAGATGAGCGACAGCATCGTCGTTCTGA
AGGTGGACGTCGACGAATGTGAGGACATCGCCACTGAGTACAACATCAACAGCATGC
CTACCTTCGTTTTCGTGAAGAACTCTAAGAAGATCGAGGAGTTCTCTGGTGCCAACG
TGGACAAGCTCCGTAACACCATCATCAAGCTCAAGCTGGCCGGCAGCGGTAGCGGTC
ACATGCACCACCACCACCACCATTCAAGCGGCGACGACGACGACAAGGTGCAGCCA
ACCGAATCAATCGTCCGCTTCCCAAACATCACTAACCTGTGCCCATTCGACGAGGTGT
TCAACGCCACCACTTTCGCCTCTGTCTACGCTTGGAACCGCAAGCGCATCTCCAACT
GTGTCGCCGACTACAGCGTGCTGTACAACTTCGCTCCTTTCTTCGCATTCAAGTGTTA
CGGAGTGTCCCCTACCAAGCTCAACGATTTGTGCTTCACAAACGTGTACGCTGACAG
CTTCGTGATCCGTGGTAACGAGGTTTCTCAAATCGCCCCTGGTCAGACAGGTAACATC
GCTGACTACAACTACAAGCTCCCAGACGACTTCACTGGCTGTGTTATCGCTTGGAACT
CAAACAAGCTGGACTCTACTGTGGGTGGTAACTACAACTACCGGTACCGTCTGTTCC
GCAAGAGCAAGCTCAAGCCATTCGAACGTGACATCAGCACAGAGATCTACCAAGCC
GGTAACAAGCCCTGCAACGGCGTTGCCGGAGTCAACTGCTACTTCCCCCTCCAGTCC
TACGGATTCCGCCCAACCTACGGCGTGGGTCACCAGCCATACCGCGTGGTTGTGCTG
AGCTTCGAACTGCTGCACGCCCCCGCTACCGTGTGTGGCCCTAAGAAGAGCACTAAC
CTCGTTAAGAACAAGTCCGTGAACTTCAACTTCAACGGTCTCTACGAAAACCAGAAG
CTGATCGCTAACCAGTTCAACTCCGCCATCGGCAAGATCCAGGACTCCCTGTCCTCCA
CAGCTTCCGCTCTGGGCAAGCTGCAGGACGTTGTGAACCAAAACGCTCAGGCCCTG
AACACACTGGTGAAGCAACTGAAGAACCACACCAGCCCCGACGTTGACCTGGGTGA
CATCAGCGGTATCAACGCTAGCGTGGTGAACATCCAGAAGGAGATCGACAGGCTGAA
CGAAGTGGCTAAGAACTTGAACGAGAGCCTCATCGACCTGCAGGAACTGTAA
SEQ ID No.27: nucleotide sequence encoding SEQ ID No.15 (construct XBB.1.5 protein) 
ATGCTGCTGGTGAACCAGAGCCACCAGGGTTTCAACAAGGAACACACTAGCAAGAT
GGTGTCTGCTATCGTCCTCTACGTGCTGCTCGCCGCCGCTGCTCACTCAGCTTTCGCC
GCTGACTCTATCCACATCAAGGACTCCGACGACCTCAAGAACCGTTTGGCCGAAGCC
GGCGACAAGCTGGTCGTTATCGACTTCATGGCTACCTGGTGTGGCCCTTGCAAGATGA
TCGGACCTAAGCTGGACGAAATGGCTAACGAGATGAGCGACAGCATCGTCGTTCTGA
AGGTGGACGTCGACGAATGTGAGGACATCGCCACTGAGTACAACATCAACAGCATGC
CTACCTTCGTTTTCGTGAAGAACTCTAAGAAGATCGAGGAGTTCTCTGGTGCCAACG
TGGACAAGCTCCGTAACACCATCATCAAGCTCAAGCTGGCCGGCAGCGGTAGCGGTC
ACATGCACCACCACCACCACCATTCAAGCGGCGACGACGACGACAAGGTGCAGCCA
ACCGAATCAATCGTCCGCTTCCCAAACATCACTAACCTGTGCCCATTCCACGAGGTGT
TCAACGCCACCACTTTCGCCTCTGTCTACGCTTGGAACCGCAAGCGCATCTCCAACT
GTGTCGCCGACTACAGCGTGATATACAACTTCGCTCCTTTCTTCGCATTCAAGTGTTAC
GGAGTGTCCCCTACCAAGCTCAACGATTTGTGCTTCACAAACGTGTACGCTGACAGC
TTCGTGATCCGTGGTAACGAGGTTTCTCAAATCGCCCCTGGTCAGACAGGTAACATCG
CTGACTACAACTACAAGCTCCCAGACGACTTCACTGGCTGTGTTATCGCTTGGAACTC
AAACAAGCTGGACTCTAAGCCGTCTGGTAACTACAACTACCTCTACCGTCTGTTCCGC
AAGAGCAAGCTCAAGCCATTCGAACGTGACATCAGCACAGAGATCTACCAAGCCGGT
AACAAGCCCTGCAACGGCGTTGCCGGACCTAACTGCTACTCTCCCCTCCAGTCCTAC
GGATTCCGCCCAACCTACGGCGTGGGTCACCAGCCATACCGCGTGGTTGTGCTGAGC
TTCGAACTGCTGCACGCCCCCGCTACCGTGTGTGGCCCTAAGAAGAGCACTAACCTC
GTTAAGAACAAGTCCGTGAACTTCAACTTCAACGGTCTCTACGAAAACCAGAAGCTG
ATCGCTAACCAGTTCAACTCCGCCATCGGCAAGATCCAGGACTCCCTGTCCTCCACA
GCTTCCGCTCTGGGCAAGCTGCAGGACGTTGTGAACCAAAACGCTCAGGCCCTGAA
CACACTGGTGAAGCAACTGAAGAACCACACCAGCCCCGACGTTGACCTGGGTGACA
TCAGCGGTATCAACGCTAGCGTGGTGAACATCCAGAAGGAGATCGACAGGCTGAACG
AAGTGGCTAAGAACTTGAACGAGAGCCTCATCGACCTGCAGGAACTGTAA
SEQ ID No.28: nucleotide sequence encoding SEQ ID No.19 (construct Mu-BA.4/5 protein) 
ATGCTGCTGGTGAACCAGAGCCACCAGGGTTTCAACAAGGAACACACTAGCAAGAT
GGTGTCTGCTATCGTCCTCTACGTGCTGCTCGCCGCCGCTGCTCACTCAGCTTTCGCC
 
GCTGACTCTATCCACATCAAGGACTCCGACGACCTCAAGAACCGTTTGGCCGAAGCC
GGCGACAAGCTGGTCGTTATCGACTTCATGGCTACCTGGTGTGGCCCTTGCAAGATGA
TCGGACCTAAGCTGGACGAAATGGCTAACGAGATGAGCGACAGCATCGTCGTTCTGA
AGGTGGACGTCGACGAATGTGAGGACATCGCCACTGAGTACAACATCAACAGCATGC
CTACCTTCGTTTTCGTGAAGAACTCTAAGAAGATCGAGGAGTTCTCTGGTGCCAACG
TGGACAAGCTCCGTAACACCATCATCAAGCTCAAGCTGGCCGGCAGCGGTAGCGGTC
ACATGCACCACCACCACCACCATTCAAGCGGCGACGACGACGACAAGGTGCAGCCA
ACCGAATCAATCGTCCGCTTCCCAAACATCACTAACCTGTGCCCATTCGACGAGGTGT
TCAACGCCACCCGTTTCGCCTCTGTCTACGCTTGGAACCGCAAGCGCATCTCCAACT
GTGTCGCCGACTACAGCGTGCTGTACAACTCAGCTTCATTCTCAGCATTCAAGTGTTA
CGGAGTGTCCCCTACCAAGCTCAACGATTTGTGCTTCACAAACGTGTACGCTGACAG
CTTCGTGATCCGTGGTAACGAGGTTTCTCAAATCGCCCCTGGTCAGACAGGTAACATC
GCTGACTACAACTACAAGCTCCCAGACGACTTCACTGGCTGTGTTATCGCTTGGAACT
CAAACAAGCTGGACTCTAAGGTGGGTGGTAACTACAACTACCGGTACCGTCTGTTCC
GCAAGAGCAACCTCAAGCCATTCGAACGTGACATCAGCACAGAGATCTACCAAGCC
GGTAACAAGCCCTGCAACGGCGTTGCCGGAGTCAACTGCTACTTCCCCCTCCAGTCC
TACGGATTCCGCCCAACCTACGGCGTGGGTCACCAGCCATACCGCGTGGTTGTGCTG
AGCTTCGAACTGCTGCACGCCCCCGCTACCGTGTGTGGCCCTAAGAAGAGCACTAAC
CTCGTTAAGAACAAGTCCGTGAACTTCAACTTCAACGGTCTCTACGAAAACCAGAAG
CTGATCGCTAACCAGTTCAACTCCGCCATCGGCAAGATCCAGGACTCCCTGTCCTCCA
CAGCTTCCGCTCTGGGCAAGCTGCAGGACGTTGTGAACCAAAACGCTCAGGCCCTG
AACACACTGGTGAAGCAACTGAAGAACCACACCAGCCCCGACGTTGACCTGGGTGA
CATCAGCGGTATCAACGCTAGCGTGGTGAACATCCAGAAGGAGATCGACAGGCTGAA
CGAAGTGGCTAAGAACTTGAACGAGAGCCTCATCGACCTGCAGGAACTGTAA
SEQ ID No.29: nucleotide sequence encoding SEQ ID No.20 (construct Mu-BQ.1.1 protein) 
ATGCTGCTGGTGAACCAGAGCCACCAGGGTTTCAACAAGGAACACACTAGCAAGAT
GGTGTCTGCTATCGTCCTCTACGTGCTGCTCGCCGCCGCTGCTCACTCAGCTTTCGCC
GCTGACTCTATCCACATCAAGGACTCCGACGACCTCAAGAACCGTTTGGCCGAAGCC
GGCGACAAGCTGGTCGTTATCGACTTCATGGCTACCTGGTGTGGCCCTTGCAAGATGA
 
TCGGACCTAAGCTGGACGAAATGGCTAACGAGATGAGCGACAGCATCGTCGTTCTGA
AGGTGGACGTCGACGAATGTGAGGACATCGCCACTGAGTACAACATCAACAGCATGC
CTACCTTCGTTTTCGTGAAGAACTCTAAGAAGATCGAGGAGTTCTCTGGTGCCAACG
TGGACAAGCTCCGTAACACCATCATCAAGCTCAAGCTGGCCGGCAGCGGTAGCGGTC
ACATGCACCACCACCACCACCATTCAAGCGGCGACGACGACGACAAGGTGCAGCCA
ACCGAATCAATCGTCCGCTTCCCAAACATCACTAACCTGTGCCCATTCGACGAGGTGT
TCAACGCCACCACTTTCGCCTCTGTCTACGCTTGGAACCGCAAGCGCATCTCCAACT
GTGTCGCCGACTACAGCGTGCTGTACAACTCCGCTTCTTTCTCCGCATTCAAGTGTTA
CGGAGTGTCCCCTACCAAGCTCAACGATTTGTGCTTCACAAACGTGTACGCTGACAG
CTTCGTGATCCGTGGTAACGAGGTTTCTCAAATCGCCCCTGGTCAGACAGGTAACATC
GCTGACTACAACTACAAGCTCCCAGACGACTTCACTGGCTGTGTTATCGCTTGGAACT
CAAACAAGCTGGACTCTACTGTGGGTGGTAACTACAACTACCGGTACCGTCTGTTCC
GCAAGAGCAAGCTCAAGCCATTCGAACGTGACATCAGCACAGAGATCTACCAAGCC
GGTAACAAGCCCTGCAACGGCGTTGCCGGAGTCAACTGCTACTTCCCCCTCCAGTCC
TACGGATTCCGCCCAACCTACGGCGTGGGTCACCAGCCATACCGCGTGGTTGTGCTG
AGCTTCGAACTGCTGCACGCCCCCGCTACCGTGTGTGGCCCTAAGAAGAGCACTAAC
CTCGTTAAGAACAAGTCCGTGAACTTCAACTTCAACGGTCTCTACGAAAACCAGAAG
CTGATCGCTAACCAGTTCAACTCCGCCATCGGCAAGATCCAGGACTCCCTGTCCTCCA
CAGCTTCCGCTCTGGGCAAGCTGCAGGACGTTGTGAACCAAAACGCTCAGGCCCTG
AACACACTGGTGAAGCAACTGAAGAACCACACCAGCCCCGACGTTGACCTGGGTGA
CATCAGCGGTATCAACGCTAGCGTGGTGAACATCCAGAAGGAGATCGACAGGCTGAA
CGAAGTGGCTAAGAACTTGAACGAGAGCCTCATCGACCTGCAGGAACTGTAA
SEQ ID No.30: nucleotide sequence encoding SEQ ID No.21 (construct Mu-XBB.1.5 protein) 
ATGCTGCTGGTGAACCAGAGCCACCAGGGTTTCAACAAGGAACACACTAGCAAGAT
GGTGTCTGCTATCGTCCTCTACGTGCTGCTCGCCGCCGCTGCTCACTCAGCTTTCGCC
GCTGACTCTATCCACATCAAGGACTCCGACGACCTCAAGAACCGTTTGGCCGAAGCC
GGCGACAAGCTGGTCGTTATCGACTTCATGGCTACCTGGTGTGGCCCTTGCAAGATGA
TCGGACCTAAGCTGGACGAAATGGCTAACGAGATGAGCGACAGCATCGTCGTTCTGA
AGGTGGACGTCGACGAATGTGAGGACATCGCCACTGAGTACAACATCAACAGCATGC
 
CTACCTTCGTTTTCGTGAAGAACTCTAAGAAGATCGAGGAGTTCTCTGGTGCCAACG
TGGACAAGCTCCGTAACACCATCATCAAGCTCAAGCTGGCCGGCAGCGGTAGCGGTC
ACATGCACCACCACCACCACCATTCAAGCGGCGACGACGACGACAAGGTGCAGCCA
ACCGAATCAATCGTCCGCTTCCCAAACATCACTAACCTGTGCCCATTCCACGAGGTGT
TCAACGCCACCACTTTCGCCTCTGTCTACGCTTGGAACCGCAAGCGCATCTCCAACT
GTGTCGCCGACTACAGCGTGATATACAACTCCGCTTCTTTCTCTGCATTCAAGTGTTAC
GGAGTGTCCCCTACCAAGCTCAACGATTTGTGCTTCACAAACGTGTACGCTGACAGC
TTCGTGATCCGTGGTAACGAGGTTTCTCAAATCGCCCCTGGTCAGACAGGTAACATCG
CTGACTACAACTACAAGCTCCCAGACGACTTCACTGGCTGTGTTATCGCTTGGAACTC
AAACAAGCTGGACTCTAAGCCGTCTGGTAACTACAACTACCTCTACCGTCTGTTCCGC
AAGAGCAAGCTCAAGCCATTCGAACGTGACATCAGCACAGAGATCTACCAAGCCGGT
AACAAGCCCTGCAACGGCGTTGCCGGACCTAACTGCTACTCTCCCCTCCAGTCCTAC
GGATTCCGCCCAACCTACGGCGTGGGTCACCAGCCATACCGCGTGGTTGTGCTGAGC
TTCGAACTGCTGCACGCCCCCGCTACCGTGTGTGGCCCTAAGAAGAGCACTAACCTC
GTTAAGAACAAGTCCGTGAACTTCAACTTCAACGGTCTCTACGAAAACCAGAAGCTG
ATCGCTAACCAGTTCAACTCCGCCATCGGCAAGATCCAGGACTCCCTGTCCTCCACA
GCTTCCGCTCTGGGCAAGCTGCAGGACGTTGTGAACCAAAACGCTCAGGCCCTGAA
CACACTGGTGAAGCAACTGAAGAACCACACCAGCCCCGACGTTGACCTGGGTGACA
TCAGCGGTATCAACGCTAGCGTGGTGAACATCCAGAAGGAGATCGACAGGCTGAACG
AAGTGGCTAAGAACTTGAACGAGAGCCTCATCGACCTGCAGGAACTGTAA
SEQ ID No.31: amino acid sequence of Delta protein precursor

MLLVNQSHQGFNKEHTSKMVSAIVLYVLLAAAAHSAFAADSIHIKDSDDLKNRLAEAG
DKLVVIDFMATWCGPCKMIGPKLDEMANEMSDSIVVLKVDVDECEDIATEYNINSMPTF
VFVKNSKKIEEFSGANVDKLRNTIIKLKLAGSGSGHMHHHHHHSSGDDDDKVQPTESIV
RFPNITNLCPFGEVFNATRFASVYAWNRKRISNCVADYSVLYNSASFSTFKCYGVSPTKLN
DLCFTNVYADSFVIRGDEVRQIAPGQTGKIADYNYKLPDDFTGCVIAWNSNNLDSKVGG
NYNYRYRLFRKSNLKPFERDISTEIYQAGSKPCNGVEGFNCYFPLQSYGFQPTNGVGYQP
YRVVVLSFELLHAPATVCGPKKSTNLVKNKSVNFNFNGLYENQKLIANQFNSAIGKIQDS

LSSTASALGKLQDVVNQNAQALNTLVKQLKNHTSPDVDLGDISGINASVVNIQKEIDRL
NEVAKNLNESLIDLQEL

The protein formed by the amino acid sequence designed based on SARS-CoV-2 Delta strains is defined as the Delta protein, the amino acid sequence is as shown in SEQ ID No.36, and the protein sequence only includes RBD and HR sequences and excludes the signal peptide, Trx, 6His tags and EK amino acid sequence.
SEQ ID No.32: nucleotide sequence encoding SEQ ID No.31 
ATGCTACTAGTAAATCAGTCACACCAAGGCTTCAATAAGGAACACACAAGCAAGATG
GTGAGCGCTATCGTGCTGTACGTCCTGCTGGCCGCTGCTGCTCACAGCGCTTTCGCTG
CTGACTCCATCCACATCAAGGACAGCGACGACCTGAAGAACCGTCTGGCCGAGGCC
GGTGACAAGCTGGTCGTCATCGACTTCATGGCCACTTGGTGCGGTCCTTGCAAGATG
ATCGGCCCTAAGCTGGACGAGATGGCTAACGAGATGTCCGACAGCATCGTGGTCCTG
AAGGTGGACGTCGACGAGTGCGAGGACATCGCCACCGAATACAACATCAACAGCAT
GCCCACCTTCGTGTTCGTGAAGAACAGCAAGAAGATCGAGGAATTTTCCGGCGCTAA
CGTCGACAAGCTGCGTAACACCATCATCAAGCTGAAGCTGGCCGGCTCCGGCTCCGG
CCACATGCATCACCACCACCACCATTCCTCCGGTGACGACGACGACAAGGTGCAGCC
AACCGAATCTATCGTCAGATTCCCAAACATCACTAACCTGTGCCCTTTCGGAGAGGTG
TTCAACGCTACCAGGTTCGCCAGCGTCTACGCTTGGAACCGCAAGCGTATCAGCAAC
TGCGTCGCCGACTACTCTGTGCTGTACAACTCCGCTAGCTTCTCTACTTTCAAGTGCT
ACGGCGTGTCACCTACCAAGCTGAACGACCTGTGCTTCACTAACGTCTACGCCGACT
CCTTCGTGATCCGCGGAGACGAAGTCCGTCAGATCGCTCCTGGACAGACCGGAAAGA
TCGCTGACTACAACTACAAGCTGCCAGACGACTTCACTGGCTGCGTGATCGCTTGGA
ACTCAAACAACCTGGACTCCAAGGTCGGTGGCAACTACAACTACCGGTACAGGCTGT
TCAGAAAGTCAAACCTGAAGCCTTTCGAGCGCGACATCTCAACCGAAATCTACCAGG
CTGGTTCCAAGCCCTGCAACGGTGTGGAGGGCTTCAACTGCTACTTCCCCCTGCAGT
CCTACGGTTTCCAGCCAACCAACGGAGTCGGTTACCAGCCTTACCGTGTGGTCGTGC
TGAGCTTCGAACTGCTCCACGCTCCTGCTACTGTGTGCGGTCCCAAGAAGTCTACTA
ACCTGGTCAAAAACAAAAGCGTCAACTTCAACTTCAACGGTCTGTACGAGAACCAG
AAGCTGATCGCTAACCAGTTCAACAGCGCCATCGGCAAGATCCAGGACTCACTGTCA
 
TCCACTGCCTCCGCTCTGGGAAAGCTGCAGGACGTCGTGAACCAGAACGCCCAGGC
TCTGAACACCCTGGTCAAGCAGCTGAAGAACCACACCTCTCCTGACGTCGACCTGGG
CGACATCAGCGGAATCAACGCTTCTGTCGTGAACATCCAGAAGGAGATCGACCGCCT
GAACGAAGTGGCCAAGAACCTGAACGAATCCCTGATTGACCTCCAAGAACTCTAA
SEQ ID No.33: The full-length amino acid sequence of Delta S antigen 
MFVFLVLLPLVSSQCVNLRTRTQLPPAYTNSFTRGVYYPDKVFRSSVLHSTQDLFLPFFSN
VTWFHAIHVSGTNGTKRFDNPVLPFNDGVYFASTEKSNIIRGWIFGTTLDSKTQSLLIVN
NATNVVIKVCEFQFCNDPFLDVYYHKNNKSWMESGVYSSANNCTFEYVSQPFLMDLEG
KQGNFKNLREFVFKNIDGYFKIYSKHTPINLVRDLPQGFSALEPLVDLPIGINITRFQTLLA
LHRSYLTPGDSSSGWTAGAAAYYVGYLQPRTFLLKYNENGTITDAVDCALDPLSETKCT
LKSFTVEKGIYQTSNFRVQPTESIVRFPNITNLCPFGEVFNATRFASVYAWNRKRISNCVA
DYSVLYNSASFSTFKCYGVSPTKLNDLCFTNVYADSFVIRGDEVRQIAPGQTGKIADYNY
KLPDDFTGCVIAWNSNNLDSKVGGNYNYRYRLFRKSNLKPFERDISTEIYQAGSKPCNG
VEGFNCYFPLQSYGFQPTNGVGYQPYRVVVLSFELLHAPATVCGPKKSTNLVKNKCVNF
NFNGLTGTGVLTESNKKFLPFQQFGRDIADTTDAVRDPQTLEILDITPCSFGGVSVITPGTN
TSNQVAVLYQGVNCTEVPVAIHADQLTPTWRVYSTGSNVFQTRAGCLIGAEHVNNSYEC
DIPIGAGICASYQTQTNSRRRARSVASQSIIAYTMSLGAENSVAYSNNSIAIPTNFTISVTTEI
LPVSMTKTSVDCTMYICGDSTECSNLLLQYGSFCTQLNRALTGIAVEQDKNTQEVFAQV
KQIYKTPPIKDFGGFNFSQILPDPSKPSKRSFIEDLLFNKVTLADAGFIKQYGDCLGDIAAR
DLICAQKFNGLTVLPPLLTDEMIAQYTSALLAGTITSGWTFGAGAALQIPFAMQMAYRFN
GIGVTQNVLYENQKLIANQFNSAIGKIQDSLSSTASALGKLQNVVNQNAQALNTLVKQL
SSNFGAISSVLNDILSRLDKVEAEVQIDRLITGRLQSLQTYVTQQLIRAAEIRASANLAAT
KMSECVLGQSKRVDFCGKGYHLMSFPQSAPHGVVFLHVTYVPAQEKNFTTAPAICHDG
KAHFPREGVFVSNGTHWFVTQRNFYEPQIITTDNTFVSGNCDVVIGIVNNTVYDPLQPEL
DSFKEELDKYFKNHTSPDVDLGDISGINASVVNIQKEIDRLNEVAKNLNESLIDLQELGK
YEQYIKWPWYIWLGFIAGLIAIVMVTIMLCCMTSCCSCLKGCCSCGSCCKFDEDDSEPV
LKGVKLHYT
SEQ ID No.34: Omicron-BA.1 S antigen full-length amino acid sequence MFVFLVLLPLVSSQCVNLTTRTQLPPAYTNSFTRGVYYPDKVFRSSVLHSTQDLFLPFFSN 
VTWFHVISGTNGTKRFDNPVLPFNDGVYFASIEKSNIIRGWIFGTTLDSKTQSLLIVNNAT
NVVIKVCEFQFCNDPFLDHKNNKSWMESEFRVYSSANNCTFEYVSQPFLMDLEGKQGN
FKNLREFVFKNIDGYFKIYSKHTPIIVRDLPQGFSALEPLVDLPIGINITRFQTLLALHRSYL
TPGDSSSGWTAGAAAYYVGYLQPRTFLLKYNENGTITDAVDCALDPLSETKCTLKSFTV
EKGIYQTSNFRVQPTESIVRFPNITNLCPFDEVFNATRFASVYAWNRKRISNCVADYSVLY
NLAPFFTFKCYGVSPTKLNDLCFTNVYADSFVIRGDEVRQIAPGQTGNIADYNYKLPDDF
TGCVIAWNSNKLDSKVSGNYNYLYRLFRKSNLKPFERDISTEIYQAGNKPCNGVAGFNC
YFPLRSYSFRPTYGVGHQPYRVVVLSFELLHAPATVCGPKKSTNLVKNKCVNFNFNGLK
GTGVLTESNKKFLPFQQFGRDIADTTDAVRDPQTLEILDITPCSFGGVSVITPGTNTSNQVA
VLYQGVNCTEVPVAIHADQLTPTWRVYSTGSNVFQTRAGCLIGAEYVNNSYECDIPIGAG
ICASYQTQTKSHRRARSVASQSIIAYTMSLGAENSVAYSNNSIAIPTNFTISVTTEILPVSMT
KTSVDCTMYICGDSTECSNLLLQYGSFCTQLKRALTGIAVEQDKNTQEVFAQVKQIYKTP
PIKYFGGFNFSQILPDPSKPSKRSFIEDLLFNKVTLADAGFIKQYGDCLGDIAARDLICAQ
KFKGLTVLPPLLTDEMIAQYTSALLAGTITSGWTFGAGAALQIPFAMQMAYRFNGIGVTQ
NVLYENQKLIANQFNSAIGKIQDSLSSTASALGKLQDVVNHNAQALNTLVKQLSSKFGAI
SSVLNDIFSRLDKVEAEVQIDRLITGRLQSLQTYVTQQLIRAAEIRASANLAATKMSECVL
GQSKRVDFCGKGYHLMSFPQSAPHGVVFLHVTYVPAQEKNFTTAPAICHDGKAHFPREG
VFVSNGTHWFVTQRNFYEPQIITTDNTFVSGNCDVVIGIVNNTVYDPLQPELDSFKEELD
KYFKNHTSPDVDLGDISGINASVVNIQKEIDRLNEVAKNLNESLIDLQELGKYEQYIKWP
WYIWLGFIAGLIAIVMVTIMLCCMTSCCSCLKGCCSCGSCCKFDEDDSEPVLKGVKLHY
T
SEQ ID No.35: full-length amino acid sequence after mutation of Omicron-BA.1 S antigen 
MFVFLVLLPLVSSQCVNLTTRTQLPPAYTNSFTRGVYYPDKVFRSSVLHSTQDLFLPFFSN
VTWFHVISGTNGTKRFDNPVLPFNDGVYFASIEKSNIIRGWIFGTTLDSKTQSLLIVNNAT
NVVIKVCEFQFCNDPFLDHKNNKSWMESEFRVYSSANNCTFEYVSQPFLMDLEGKQGN
FKNLREFVFKNIDGYFKIYSKHTPIIVRDLPQGFSALEPLVDLPIGINITRFQTLLALHRSYL
TPGDSSSGWTAGAAAYYVGYLQPRTFLLKYNENGTITDAVDCALDPLSETKCTLKSFTV
EKGIYQTSNFRVQPTESIVRFPNITNLCPFDEVFNATRFASVYAWNRKRISNCVADYSVLY
NSASFSTFKCYGVSPTKLNDLCFTNVYADSFVIRGDEVRQIAPGQTGNIADYNYKLPDDF
TGCVIAWNSNKLDSKVSGNYNYLYRLFRKSNLKPFERDISTEIYQAGNKPCNGVAGFNC
YFPLRSYSFRPTYGVGHQPYRVVVLSFELLHAPATVCGPKKSTNLVKNKCVNFNFNGLK
GTGVLTESNKKFLPFQQFGRDIADTTDAVRDPQTLEILDITPCSFGGVSVITPGTNTSNQVA
VLYQGVNCTEVPVAIHADQLTPTWRVYSTGSNVFQTRAGCLIGAEYVNNSYECDIPIGAG
 
ICASYQTQTKSHRRARSVASQSIIAYTMSLGAENSVAYSNNSIAIPTNFTISVTTEILPVSMT
KTSVDCTMYICGDSTECSNLLLQYGSFCTQLKRALTGIAVEQDKNTQEVFAQVKQIYKTP
PIKYFGGFNFSQILPDPSKPSKRSFIEDLLFNKVTLADAGFIKQYGDCLGDIAARDLICAQ
KFKGLTVLPPLLTDEMIAQYTSALLAGTITSGWTFGAGAALQIPFAMQMAYRFNGIGVTQ
NVLYENQKLIANQFNSAIGKIQDSLSSTASALGKLQDVVNHNAQALNTLVKQLSSKFGAI
SSVLNDIFSRLDKVEAEVQIDRLITGRLQSLQTYVTQQLIRAAEIRASANLAATKMSECVL
GQSKRVDFCGKGYHLMSFPQSAPHGVVFLHVTYVPAQEKNFTTAPAICHDGKAHFPREG
VFVSNGTHWFVTQRNFYEPQIITTDNTFVSGNCDVVIGIVNNTVYDPLQPELDSFKEELD
KYFKNHTSPDVDLGDISGINASVVNIQKEIDRLNEVAKNLNESLIDLQELGKYEQYIKWP
WYIWLGFIAGLIAIVMVTIMLCCMTSCCSCLKGCCSCGSCCKFDEDDSEPVLKGVKLHY
T
SEQ ID No.36: amino acid sequence of Delta protein 
VQPTESIVRFPNITNLCPFGEVFNATRFASVYAWNRKRISNCVADYSVLYNSASFSTFKCY
GVSPTKLNDLCFTNVYADSFVIRGDEVRQIAPGQTGKIADYNYKLPDDFTGCVIAWNSN
NLDSKVGGNYNYRYRLFRKSNLKPFERDISTEIYQAGSKPCNGVEGFNCYFPLQSYGFQP
TNGVGYQPYRVVVLSFELLHAPATVCGPKKSTNLVKNKSVNFNFNGLYENQKLIANQFN
SAIGKIQDSLSSTASALGKLQDVVNQNAQALNTLVKQLKNHTSPDVDLGDISGINASVV
NIQKEIDRLNEVAKNLNESLIDLQEL
SEQ ID No.37: amino acid sequence of RBD_{BA.1} -HR protein precursor 
MLLVNQSHQGFNKEHTSKMVSAIVLYVLLAAAAHSAFAADSIHIKDSDDLKNRLAEAG
DKLVVIDFMATWCGPCKMIGPKLDEMANEMSDSIVVLKVDVDECEDIATEYNINSMPTF
VFVKNSKKIEEFSGANVDKLRNTIIKLKLAGSGSGHMHHHHHHSSGDDDDKVQPTESIV
RFPNITNLCPFDEVFNATRFASVYAWNRKRISNCVADYSVLYNLAPFFTFKCYGVSPTKL
NDLCFTNVYADSFVIRGDEVRQIAPGQTGNIADYNYKLPDDFTGCVIAWNSNKLDSKVS
GNYNYLYRLFRKSNLKPFERDISTEIYQAGNKPCNGVAGFNCYFPLRSYSFRPTYGVGHQ
PYRVVVLSFELLHAPATVCGPKKSTNLVKNKSVNFNFNGLYENQKLIANQFNSAIGKIQD
SLSSTASALGKLQDVVNQNAQALNTLVKQLKNHTSPDVDLGDISGINASVVNIQKEIDRL
NEVAKNLNESLIDLQEL
SEQ ID No.38: nucleotide sequence encoding SEQ ID No.37 
ATGCTGCTGGTGAACCAGAGCCACCAGGGTTTCAACAAGGAACACACTAGCAAGAT
GGTGTCTGCTATCGTCCTCTACGTGCTGCTCGCCGCCGCTGCTCACTCAGCTTTCGCC
GCTGACTCTATCCACATCAAGGACTCCGACGACCTCAAGAACCGTTTGGCCGAAGCC
GGCGACAAGCTGGTCGTTATCGACTTCATGGCTACCTGGTGTGGCCCTTGCAAGATGA
TCGGACCTAAGCTGGACGAAATGGCTAACGAGATGAGCGACAGCATCGTCGTTCTGA
AGGTGGACGTCGACGAATGTGAGGACATCGCCACTGAGTACAACATCAACAGCATGC
CTACCTTCGTTTTCGTGAAGAACTCTAAGAAGATCGAGGAGTTCTCTGGTGCCAACG
TGGACAAGCTCCGTAACACCATCATCAAGCTCAAGCTGGCCGGCAGCGGTAGCGGTC
ACATGCACCACCACCACCACCATTCAAGCGGCGACGACGACGACAAGGTGCAGCCA
ACCGAATCAATCGTCCGCTTCCCAAACATCACTAACCTGTGCCCATTCGACGAGGTGT
TCAACGCCACCCGTTTCGCCTCTGTCTACGCTTGGAACCGCAAGCGCATCTCCAACT
GTGTCGCCGACTACAGCGTGCTGTACAACCTTGCTCCTTTCTTCACCTTCAAGTGTTA
CGGAGTGTCCCCTACCAAGCTCAACGATTTGTGCTTCACAAACGTGTACGCTGACAG
CTTCGTGATCCGTGGTGACGAGGTTCGCCAAATCGCCCCTGGTCAGACAGGTAACAT
CGCTGACTACAACTACAAGCTCCCAGACGACTTCACTGGCTGTGTTATCGCTTGGAA
CTCAAACAAGCTGGACTCTAAGGTGTCTGGTAACTACAACTACCTCTACCGTCTGTTC
CGCAAGAGCAACCTCAAGCCATTCGAACGTGACATCAGCACAGAGATCTACCAAGCC
GGTAACAAGCCCTGCAACGGCGTTGCCGGATTCAACTGCTACTTCCCCCTCCGTTCCT
ACTCATTCCGCCCAACCTACGGCGTGGGTCACCAGCCATACCGCGTGGTTGTGCTGA
GCTTCGAACTGCTGCACGCCCCCGCTACCGTGTGTGGCCCTAAGAAGAGCACTAACC
TCGTTAAGAACAAGTCCGTGAACTTCAACTTCAACGGTCTCTACGAAAACCAGAAGC
TGATCGCTAACCAGTTCAACTCCGCCATCGGCAAGATCCAGGACTCCCTGTCCTCCAC
AGCTTCCGCTCTGGGCAAGCTGCAGGACGTTGTGAACCAAAACGCTCAGGCCCTGA
ACACACTGGTGAAGCAACTGAAGAACCACACCAGCCCCGACGTTGACCTGGGTGAC
ATCAGCGGTATCAACGCTAGCGTGGTGAACATCCAGAAGGAGATCGACAGGCTGAAC
GAAGTGGCTAAGAACTTGAACGAGAGCCTCATCGACCTGCAGGAACTGTAA
SEQ ID No.39: amino acid sequence of RBD_{BAa.1} -HR protein

VQPTESIVRFPNITNLCPFDEVFNATRFASVYAWNRKRISNCVADYSVLYNLAPFFTFKCY
GVSPTKLNDLCFTNVYADSFVIRGDEVRQIAPGQTGNIADYNYKLPDDFTGCVIAWNSN

KLDSKVSGNYNYLYRLFRKSNLKPFERDISTEIYQAGNKPCNGVAGFNCYFPLRSYSFRP
TYGVGHQPYRVVVLSFELLHAPATVCGPKKSTNLVKNKSVNFNFNGLYENQKLIANQFN
SAIGKIQDSLSSTASALGKLQDVVNQNAQALNTLVKQLKNHTSPDVDLGDISGINASVV
NIQKEIDRLNEVAKNLNESLIDLQEL

Wherein, in the above-mentioned amino acid sequences, HR1 and HR2 sequences of the S protein are sequence segments taking "LYENQKL" as beginnings and taking "IDLQEL" as terminals.

Beneficial effects: firstly, based on the SARS-CoV-2 variant strain Delta, RBD sequence or mutated RBD sequence in S protein of Omicron variant subline BA.4/5, BQ.1.1, BA.1 and XBB.1.5 and heptad repeat regions HR1 and HR2, the present invention can spontaneously form a trimer for protein recombination, and protein construction is carried out on the basis of full-length S protein sequence of Delta, BA.4/5, BQ.1.1, BA.1 and XBB.1.5, to prepare multiple recombinant proteins and be made into vaccines for resisting the infection by different SARS-CoV-2 variant strains.

In the present invention, different recombinant proteins are combined and added with the adjuvant to be prepared into polyvalent vaccines such as divalent, trivalent and tetravalent vaccines, animal experiments have proved that the recombinant proteins RBD_{Delta}-HR, RBD_{BA.5}-HR and RBD_{XBB.1.5}-HR prepared on the basis of the Delta and Omicron variant subline BA.4/5, the RBD sequence of XBB.1.5 and heptad repeat regions HR1 and HR2 are Delta protein, BA.5 protein and XBB.1.5 protein, respectively, and the trivalent vaccine prepared by combining the Delta protein, BA.5 protein and XBB.1.5 protein and adding the adjuvant can significantly enhance the protective efficiency of the vaccine, increase the titer of the neutralizing antibody and the ability to resist virus infection, and has broad spectrum, particularly has better prevention and treatment effect on the cross infection caused by Omicron and the subtype thereof.

### BRIEF DESCRIPTION OF THE DRAWINGS

FIG. 1 is a design graph of pFastBac1-GP67-Trx-His-EK-S-RBD(Omicron_XBB.1.5)-HR in Embodiment 1;
FIG. 2 is a 1% agarose gel electrophoresis image of PCR products from clones 1-3# in Embodiment 1;
FIG. 3 is a PCR-identified 1% agarose gel electrophoresis graph of recombinant bacmid in Embodiment 1;
FIG. 4 is a packaging and amplification flowchart of a recombinant baculovirus in Embodiment 1;
FIG. 5 is a WB verification result graph during baculovirus expansion in Embodiment 1;
FIG. 6 is a result graph of eluting supernatant after baculovirus infection through a Ni-affinity chromatography in Embodiment 1;
FIG. 7 is a result graph of verifying an enzyme digestion effect by an EK enzyme digestion sample in Embodiment 1;
FIG. 8 is a graph of packing purification and SDS-PAGE identification through a Ni-affinity chromatography after EK enzyme digestion in Embodiment 1;
FIG. 9 is a result graph of IgG in the serum of immunized mice by different vaccines in Embodiment 3;
FIG. 10 is a result graph of neutralizing antibodies in the serum of mice by the combination of multiple vaccines in Embodiment 4;
FIG. 11 is a result graph of neutralizing antibodies in the serum of mice showing a RBD_{WT} monovalent vaccine, a RBD_{XBB.1.5} monovalent vaccine and a bivalent vaccine (RBD_{WT}+RBD_{XBB.1.5}) mixed in different ratios in Embodiment 4;
FIG. 12 is a result graph of neutralizing antibodies in the serum of immunized mice by RBD_{Delta-}HR, RBD_{BA.5}-HR, RBD_{XBB.1.5}-HR monovalent vaccines and a Tri-Vac vaccine mixed in different ratios in Embodiment 4;
FIG. 13 is a result graph of neutralizing antibodies in the serum of immunized mice with 1.67µg and 10µg of RBD_{Delta}-HR, RBD_{BA.5}-HR, RBD_{XBB.1.5}-HR, 10µg of Tri-Vac vaccine mixed at 1:1:4 in Embodiment 4;
FIG. 14 is a result graph of neutralizing antibodies of pseudovirus in the serum of sequential mice of a trivalent vaccine (RBD_{Delta}-HR+RBD_{BA.5}-HR+RBD_{XBB.1.5}-HR) mixed at 1:1:4 in Embodiment 4;
FIG. 15 is a result graph of neutralizing antibodies in the serum of sequential mice by a bivalent vaccine mixed at different ratios in Embodiment 4;
FIG. 16 is a result graph of neutralizing antibodies in the serum of sequential immunized mice by a monovalent vaccine RBD_{XBB.1.5} and a bivalent vaccine (RBD_{WT}+RBD_{XBB.1.5}, with a mixing ratio of 1:3) in Embodiment 4;
FIG. 17 is a result graph of neutralizing antibodies in the serum of sequential immunized mice by a monovalent vaccine RBD_{XBB.1.5}- and a trivalent vaccine (with a mixing ratio of 1:1:4) in Embodiment 4;
FIG. 18 is a result graph of neutralizing antibodies of euvirus in the serum of Tri-Vac mice mixed at 1:1:4 in Embodiment 5;
FIG. 19 is a result graph of neutralizing antibodies of euvirus in the serum of Tri-Vac sequential mice mixed at 1:1:4 in Embodiment 5;
FIG. 20 is a result graph of neutralizing antibodies of euvirus in the serum of sequential mice by a bivalent vaccine mixed at 1:3 in Embodiment 5;
FIG. 21 is a challenge experiment result graph of immunized mice by different doses of a trivalent vaccine Tri-Vac mixed at 1:1:4 in Embodiment 6; and
FIG. 22 is a challenge experiment result graph of live virus of sequential immunized mice by a bivalent vaccine (RBD_{WT}+ RBD_{XBB.1.5}, with a mixing ratio of 1:3) in Embodiment 6.

### Embodiments

### Terms and abbreviations:

Monophosphate lipid A (MPL); squalene-based oil-in-water emulsion (MF59), recombinant cholera toxin (rCTB), astragalus polysaccharide (APS), phosphatidylethanolamine (PE), phosphatidylcholine (PC), cholesterol (Chol), dioleoyl phosphatidylethanolamine (DOPE)(2,3-dioleoxypropyl) trimethyl ammonium chloride (DOTAP), N-[N- trimethylamine chloride (DOTMA), cationic cholesterol (DC-Chol), dimethyl trifluoroacetate-2,3-dioleoxypropyl-2-(2-spermamido) ethylammonium (DOSPA), trimethyl dodecyl ammonium bromide (DTAB), trimethyl tetradecyl ammonium bromide (TTAB), trimethyl cetylammonium bromide (CTAB), dimethyl didoctadecyl ammonium bromide (DDAB), CpG ODN (A nucleotide sequence with unmethylated cytosine and guanine dinucleotides as the core sequence, artificially synthesized CpG).

The present invention provides a protein, monovalent vaccine and polyvalent vaccine against infection by a SARS-CoV-2 Omicron variant strain and subtype thereof, which mainly induce immune responses such as the production of antibodies in vivo and block the binding of the S protein in SARS-CoV-2 to the ACE2 receptor of the host cell for the S protein of the SARS-CoV-2 virus, specifically the ACE2 receptor-binding domain blocking the S protein, thus helping the host resist the infection of the coronavirus, particularly having a better prevention and treatment effect on the cross infection caused by SARS-CoV-2 or mutated viruses thereof, for example, cross infection caused by SARS-CoV-2 or Delta variants, Omicron variant subline BA.5, BQ.1.1, XBB.1.5, etc.

The solution of the present invention will be explained with reference to embodiments. It will be understood by those skilled in the art that the following examples are merely intended for illustrating the present invention, instead of limiting the scope of the present invention. If specific technologies or conditions are not specified in the embodiments, technologies or conditions described in literatures in the art or product specifications should prevail. If manufacturers are not indicated in reagents or instruments used, they are all conventional products that can be obtained through market purchase.

### Embodiment 1: preparation of recombinant protein through systematic expression of insect baculovirus (taking S-RBD_{XBB.1.5}-HR as an example)

### 1. Construction and design of S-RBD_{XBB.1.5}-HR

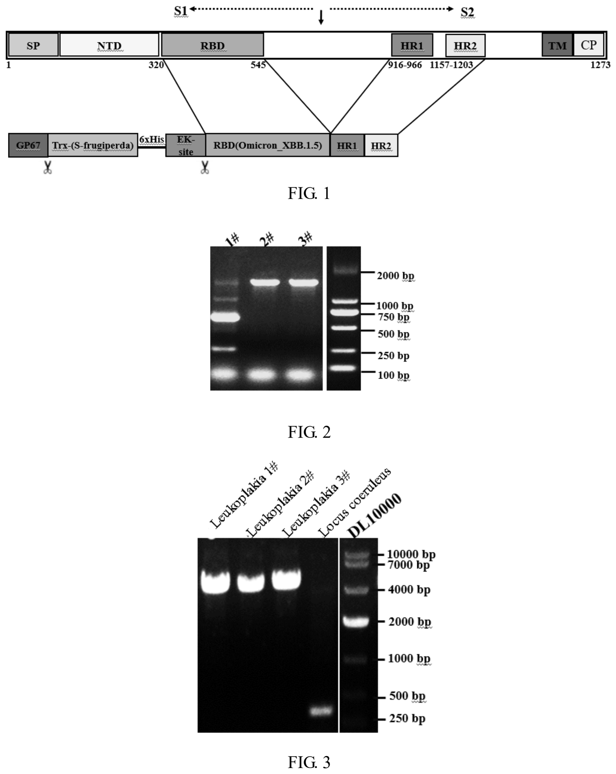

The S protein of SARS-CoV-2 was a membrane-located protein, therefore, in order to mimic its secretion process, the signal peptide sequence of GP67 was added to the N-terminal of the protein during the construction of S-RBD(Omicron_XBB.1.5)-HR protein expression in SARS-CoV-2 to assist in the secretion and expression of the protein, and this signal peptide would be spontaneously removed by insect cells in a protein secretion process; meanwhile, thioredoxin (Trx) tag of *Spodoptera frugiperda* (S. frugiperda) was added after GP67 signal peptide to assist in S-RBD(Omicron_XBB.1.5)-HR folding, 6xhis tag was added to help subsequent purification, and an EK enzyme digestion site was added to remove both Trx and 6xhis tags. The construction and design of protein expression will be able to remove all non-S-RBD(Omicron_XBB.1.5)-HR redundant amino acids through EK enzyme digestion. The mode constructed and designed through the expression was as shown in FIG. 1, that is, the amino acid sequence of GP67-Trx-His-EK-S-RBD(Omicron_XBB.1.5)-HR was as shown in SEQ ID No.15, and the nucleotide sequence encoding SEQ ID No.15 was as shown in SEQ ID No.27. The construction of the above sequence was on the basis of the 320-545 sites of RBD sequence of S protein in the SARS-CoV-2 Omicron variant strain XBB.1.5, in which amino acids at sites 52, 54 and 56 were F, P and F, respectively.

### 2. Identification for construction of recombinant plasmid

The signal peptide-Trx-6His-EK-RBD-HR1-HR2 amino acid sequence of XBB.1.5, encoded by SEQ ID No.15, was cloned into the pFastBac1 vector plasmid as the designed encoding fragment, and was subjected to identification via PCR of the bacterial solution. The identification of the bacteria solution PCR showed that in the three selected clones, 2# and 3# clones expanded GP67-Trx-His-EK-S-RBD(Omicron_XBB.1.5)-HR fragments successfully, as shown in FIG. 2.

### 3. Identification of recombinant bacmid

The correctly identified pFastBac1-GP67-Trx-His-EK-S-RBD(Omicron_XBB.1.5)-HR recombinant clone was selected, after extracting the recombinant plasmid, the DH10B competent cell was transformed, and identification of blue-white-spot bacteria solution PCR was performed. The bacteria solution PCR product was detected by 1% agarose gel electrophoresis, and the identification result was as shown in FIG. 3. The white spot showed the bacmid clone that had been recombined, and the blue spot showed the bacmid clone that had not been recombined.

### 4. Packaging of recombinant baculovirus

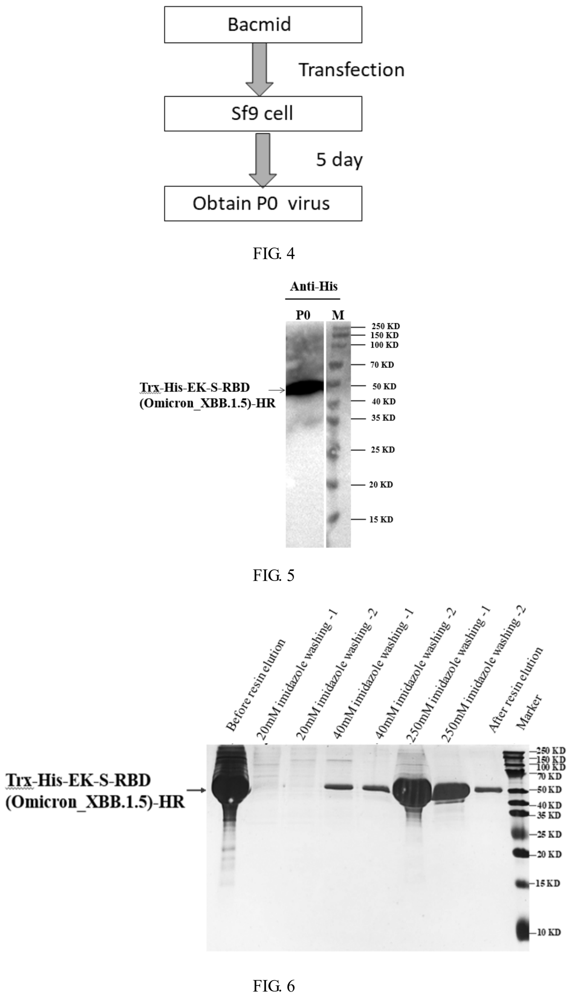

The recombinant bacmid was transfected into sf9 insect cells, and P0-passage recombinant baculovirus was harvested after 5 days. The packaging and amplification flowchart of the recombinant baculovirus were as shown in FIG. 4.

### 5. Expression and verification of target protein

The expression of the target protein simultaneously took place along with the amplification of the aforementioned baculovirus. While before removing the tag of the target protein, His tag was included, and therefore we used the WB experiment of Anti-His to verify the expression of the recombinant protein. The verification result showed that an obvious stripe was observed between 40 KD-55 KD Marker stripes, and had a size matching with that of the Trx-His-EK-S-RBD(Omicron_XBB.1.5)-HR protein, indicating the successful amplification of baculovirus and successful expression of the target protein. The testing result was as shown in FIG. 5.

### 6. Purification and identification of target protein

The P0-passage recombinant baculovirus was infected with sf9 cells, a cell culture fluid was harvested after 3 days, and the verification for the protein purification was carried out through Ni-affinity chromatography packing. The result was as shown in FIG. 6. The target protein was mainly eluted under 40 mM and 250 mM imidazoles, and the target protein with a high purity might be obtained after elution.

### 7. Verification of tag removal by EK enzyme digestion

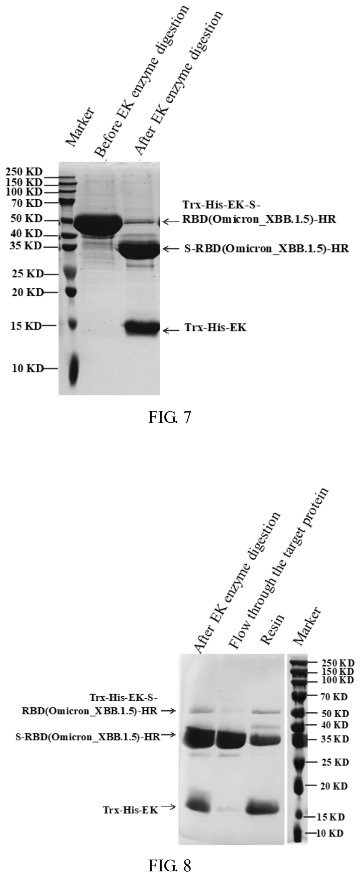

The eluent for the target protein was concentrated and adjusted to a concentration of 1 mg/mL, an EK protease was added, and after 14 hours of digestion by the enzyme at 18 °C, the identification was performed through SDS-PAGE gel electrophoresis. The result was as shown in FIG. 7. The result showed that the EK enzyme could remove the Trx-His-EK (the amino acid sequence at the EK enzyme digestion site) tag from the target protein.

### 8. Removal verification for the removed tag

The sample after EK enzyme digestion was reversely hung for 10 min by using the Ni-affinity chromatography packing, and the sample was eluted by using 250 mM imidazole after flowing through. The Trx-His-EK-S-RBD(Omicron_XBB.1.5)-HR protein (uncut and complete protein) and S-RBD(Omicron_XBB.1.5)-HR protein were separated from the cut Trx-His-EK tag. The result was as shown in FIG. 8. The result showed that the Trx-His-EK tag could be removed through a reverse hanging subsequent purification method of the Ni-affinity chromatography packing after being cut, thus the S-RBD (Omicron_XBB.1.5)-HR protein excluding any tag was obtained and defined as the recombinant protein RBD_{XBB.1.5}-HR, namely, XBB.1.5 protein, with a protein amino acid sequence as shown in SEQ ID No.3.

Similarly, with reference to the above-method construction method for the recombinant protein RBD_{XBB.1.5}-HR.

The recombinant protein RBD_{XBB.1.5} was constructed, the HR1 and HR2 sequences of the amino acid sequence of GP67-Trx-His-EK-S-RBD(Omicron_XBB.1.5) were removed from the amino acid sequence as shown in SEQ ID No.15, and the HR1 and HR2 sequences of the amino acid sequence of the recombinant protein were removed from the amino acid sequence as shown in SEQ ID No.3.

The recombinant protein RBD_{BA.5}-HR was constructed, namely, BA.5 protein, the amino acid sequence of GP67-Trx-His-EK-S-RBD(Omicron BA.5)-HR was as shown in SEQ ID No.13, the nucleotide sequence encoding its amino acid sequence was shown in SEQ ID No.25, and the amino acid sequence of the recombinant protein was as shown in SEQ ID No.1. The construction of RBD_{BA.5}-HR was on the basis of the 320-545 sites of RBD sequence of S protein in the SARS-CoV-2 Omicron variant strain BA.4/5, in which amino acids at sites 52, 54 and 56 were F, P and F, respectively.

The recombinant protein RBD_{Delta}-HR was constructed, namely, the Delta protein, the amino acid sequence of GP67-Trx-His-EK-S-RBD(Delta)-HR was as shown in SEQ ID No.31, the nucleotide sequence encoding its amino acid sequence was as shown in SEQ ID No.32, and the amino acid sequence of the recombinant protein was as shown in SEQ ID No.36.

The recombinant protein RBD_{BA.1}-HR was constructed, the amino acid sequence of GP67-Trx-His-EK-S-RBD(Omicron_BA.1)-HR was as shown in SEQ ID No.37, the nucleotide sequence encoding its amino acid sequence was shown in SEQ ID No.38, and the amino acid sequence of the recombinant protein was as shown in SEQ ID No.39.

The recombinant protein RBD_{WT}-HR was constructed. The RBD in the amino acid sequence of GP67-Trx-His-EK-S-RBD (original strain)-HR was the amino acid sequence spanning sites 320-545 of the S protein of the SARS-CoV-2 original strain, and the rest of the amino acid sequence of the protein, as well as its construction method, were consistent with those of RBD_{XBB.1.5}-HR..

Similarly, the recombinant protein RBD_{WT} is constructed, with the RBD sequence of the protein consisting of amino acids sites 320 - 545 from the S protein of the original SARS-CoV-2 strain, while the remaining protein sequences and construction methods are consistent with those of RBD_{XBB.1.5}.

Through the above-mentioned method, the applicant successfully expressed and constructed the recombinant protein RBD_{XBB.1.5}-HR (with an amino acid sequence as shown in SEQ ID No.3), RBD_{XBB.1.5}, RBD_{Delta}-HR (with an amino acid sequence as shown in SEQ ID No.36), RBD_{BA.5}-HR (with an amino acid sequence as shown in SEQ ID No.1), RBD_{BA.1}-HR (with an amino acid sequence as shown in SEQ ID No.39), RBD_{WT}-HR and RBD_{WT}, and the monovalent vaccine was prepared by the expressed recombinant protein by using diluting with PBS buffer and adding adjuvant separately or the polyvalent vaccine was prepared by the combined recombinant protein, for subsequent research such as animal immunization.

### Embodiment 2: animal preparation, mice immunization and sample collection

1. Immunization procedure of monovalent vaccine: female NIH mice aged 6-8 weeks were purchased from Charles River Laboratories, and the mice were raised in a specific pathogen-free environment at the National Key Laboratory of Biotherapy, Sichuan University. The recombinant protein was mixed with MF59 adjuvant at an equal volume ratio, the mice were subjected to intramuscular immunization for three times at days 0, 14, 28, administering 10 ug protein was administrated to each mouse at each time, the blood of the mice was collected at the 7^{th} day after three immunizations to test neutralizing antibodies, and the immune effect of the vaccine was evaluated.
2. Immunization procedure of bivalent vaccine: female NIH mice aged 6-8 weeks were purchased from Charles River Laboratories, and the mice were raised in an environment without special pathogens of national key laboratory for biotherapy in Sichuan University. Two kinds of different recombinant proteins were mixed with an equal volume ratio of adjuvant MF59 mixed in a specific mass ratio, the mice were intramuscularly immunized three times at days 0, 14, 28, 10 ug protein was administrated to each mouse at each time, the blood of the mice was collected at the 7^{th} day after three immunizations to test neutralizing antibodies, and the immune effect of the vaccine was evaluated.
3. Immunization procedure of trivalent vaccine: female NIH mice aged 6-8 weeks were purchased from Charles River Laboratories, and the mice were raised in an environment without special pathogens of national key laboratory for biotherapy in Sichuan University. Three kinds of different recombinant proteins were mixed with an equal volume ratio of adjuvant MF59 after being mixed at a certain mass ratio, the mice were subjected to intramuscular immunization for three times at days 0, 14, 28, 10 ug protein was administrated to each mouse at each time, the blood of the mice was collected at the 7^{th} day after three immunizations to test neutralizing antibodies, and the immune effect of the vaccine was evaluated. Wherein, RBD_{Delta}-HR+RBD_{BA.5}-HR+RBD_{XBB.1.5}-HR trivalent vaccine was named as "Tri-Vac" for short.
4. Basic immunization procedure of quadrivalent vaccine: female NIH mice aged 6-8 weeks were purchased from Charles River Laboratories, and the mice were raised in an environment without special pathogens of national key laboratory for biotherapy in Sichuan University. Four kinds of different recombinant protein were mixed with an equal volume ratio of adjuvant MF59 after being mixed at a certain mass ratio, the mice were subjected to intramuscular immunization for three times at days 0, 14, 28, 10 ug protein was administrated to each mouse at each time, the blood of the mice was collected at the 7^{th} day after three immunizations to test neutralizing antibodies, and the immune effect of the vaccine was evaluated.
5. Sequential immunization procedure: female NIH mice aged 6-8 weeks were purchased from Charles River Laboratories, and the mice were raised in an environment without special pathogens of national key laboratory for biotherapy in Sichuan University. Each mouse was subjected to intramuscular immunization of 25U inactivated vaccine (IV for short, produced by China National Pharmaceutical Group Co., Ltd.) for three times at days 0, 14, 42, the sequential reinforcement and intramuscular injection for the inactivated vaccine and recombinant protein vaccine were separately carried out after the last immunization, the immunization dose was 10ug per mouse, and serums were collected at the 14^{th} day after reinforcement to detect the neutralizing antibodies of pseudovirus.

### Embodiment 3: detection of antibodies by an enzyme-linked immunosorbent assay (ELISA)

To detect anti-SARS-CoV-2 RBD-specific IgG and IgA, 96-well plates (NUNC-MaxiSorp, Thermo Fisher Scientific) were coated with 1µg/mL of recombinant RBD protein in carbonate-bicarbonate buffer and incubated overnight at 4°C . In the next day, the plate was washed three times with 1×PBS containing 0.1% Tween-20 (PBST), and then sealed for one hour with PBST containing 1%BSA. The continuously diluted serum, broncho-pulmonary lavage or nose swab in the dilution buffer solution were added to the well (100 µl/well). After the incubation was performed for one hour at 37°C, the plate was washed for three times, then diluted horse radish peroxidase (HRP) conjugated goat anti-mouse IgG (1:10000, southern biotech, Cat: 0107-05), HRP-conjugated goat anti-mouse IgG (1:5000), HRP-conjugated goat anti-rabbit IgG, HRP-conjugated goat anti-rabbit IgG, HRP-conjugated goat anti-human IgG (southern biotech, Cat: 62-8420) or HRP-conjugated goat anti-human IgA (southern biotech, Cat: 2050-05). Then, after the incubation was performed for one hour at 37°C, the plate was further washed three times, and developed for 10 min with 3,3',5,5'-tetramethylbenzidine (TMB) at room temperature. Such incubation was stopped with 1 M H₂SO₄. Finally, the absorbance at 450 nm was measured on a microwell plate reader (Spectramax ABS, Molecular Devices). A terminal titer was defined as the highest inverse dilution of serum with the absorbance ≥2.1 times of negative control serum value.

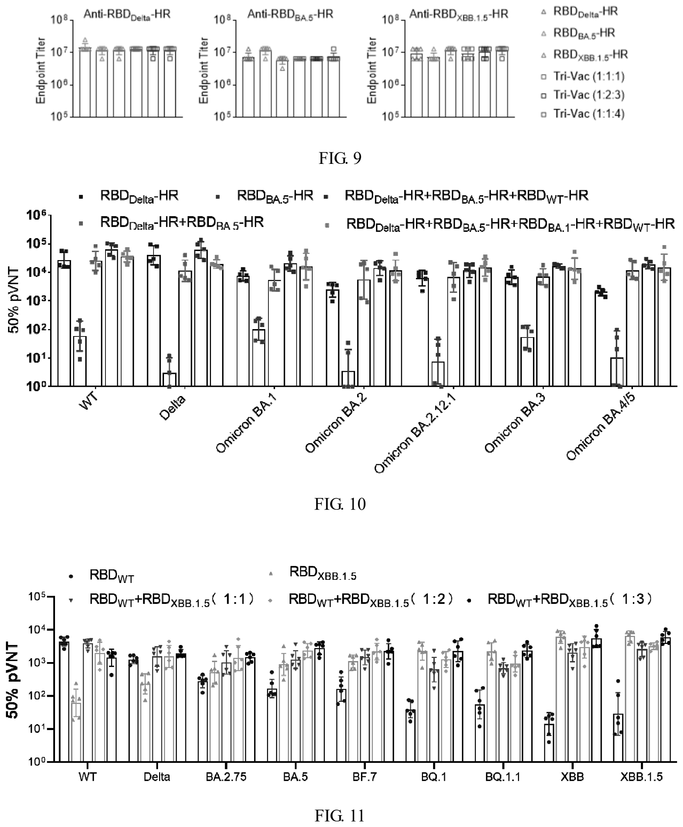

The result was as shown in FIG. 9, after completing three-dose immunization, all the monovalent RBD_{Delta}-HR, RBD_{BA.5}-HR and RBD_{XBB.1.5}-HR, recombinant protein vaccine and trivalent vaccine Tri-Vac (mixing at different ratios according to the sequence of RBD_{Delta}-HR, RBD_{BA.5}-HR and RBD_{XBB.1.5}-HR) could induce high-level IgG; and IgG generated for RBD_{Delta}-HR and RBD_{XBB.1.5}-HR by the separate RBD_{XBB.1.5}-HR vaccine and trivalent vaccine Tri-Vac was obviously higher than that for RBD_{BA.5}-HR, indicating that the trivalent vaccine Tri-Vac might generate stronger humoral immunity protective capability for RBD_{XBB.1.5}-HR.

### Embodiment 4: pseudovirus neutralization test for SARS-CoV-2

To detect the titer of neutralizing antibodies in serum and bronchoalveolar lavage fluid (BALF) samples, the pseudovirus neutralization test was carried out as mentioned above. The pseudovirus of luciferase was expressed, including precursor, δ and Omicron subline (BA.2.75, BA.5, BF.7, BQ.1, BQ.1.1, XBB, XBB.1.5, XBB.1.16 and the like), purchased from Genomeditech Company.

In short, the inactivated serum and the BALF sample (30 min, 56°C) were diluted by three times, with a range of 30-65610, and then the diluted inactivated serum and BALF sample were incubated with the equal volume and different dilutions of pseudovirus for one hour at 37°C. Then, 1.2×10⁴ HEK-293T cells expressing human ACE2 receptor (293T/ACE2) were added in each well, and were incubated for 48 hours at 37°C to express the luciferase. Finally, the supernate was removed, then a lysis reagent (Beyotime, RG005) with a luciferase substrate was added, and a multi-mode microwell plate reader (PerkinElmer, USA) was used to measure the luminous quantity in the 293T/ACE2 cell. 50% neutralizing rate of the pseudovirus was measured and calculated with GraphPad Prism 8.0.2. A positive control group included cells and viruses, a negative control group only included cells, and a sample group included cells, samples and viruses. A neutralizing percentage was calculated by the following formulas: Neutralization (%) = (positive sample-sample to be measured/positive sample-negative sample) × 100%

RBD_{Delta}-HR, RBD_{BA.5}-HR, RBD_{BA.1}-HR and RBD_{WT}-HR proteins constituted the monovalent vaccine, the bivalent vaccine (with a mixing ratio of 1:1), the trivalent vaccine (with a mixing ratio of 1:1:1) and the quadrivalent vaccine (with a mixing ratio of 1:1:1:1) that immunize the mouse, respectively, and serums were taken to measure the neutralizing antibody level to WT, Delta, BA.1, BA.2, BA.2.12.1, BA.3 and BA.4/5 viruses. As shown in FIG. 10, the pure RBD_{Delta}-HR as well as the bivalent, trivalent and quadrivalent vaccines prepared therefrom all generate stronger neutralizing antibodies, and the antibody level of the polyvalent vaccine was obviously higher than that of the pure protein vaccine. It has showed that the polyvalent vaccine can provide better protection against the original strain and the Omicron variant strain virus.

Monovalent vaccines consisting of FBD_{WT}, RBD_{XBB.1.5} protein and bivalent vaccines consisting of RBD_{WT}+RBD_{XBB.1.5} proteins (with different mixing ratios) were used to immunize mice, and serums were collected to assess the levels of neutralizing antibodies against WT, Delta, BA.2.75, BA.5, BF.7, BQ.1, BQ.1.1, XBB, and XBB.1.5 viruses.. As shown in FIG. 11, the pure RBD_{WT} protein vaccine had strong protection against the original strain and the Delta strain virus, but had weak protection against the Omicron variant strain virus; RBD_{XBB.1.5} vaccine had strong protection against the Omicron variant strain virus, but had weak protection against the original strain and the Delta strain virus; however, no matter how large the mixing ratio is, the RBD_{WT}+RBD_{XBB.1.5} bivalent vaccine had strong protection against the original strain, Delta strain and Omicron variant strain virus, showing the unique advantage of the RBD_{WT}+RBD_{XBB.1.5} bivalent vaccine.

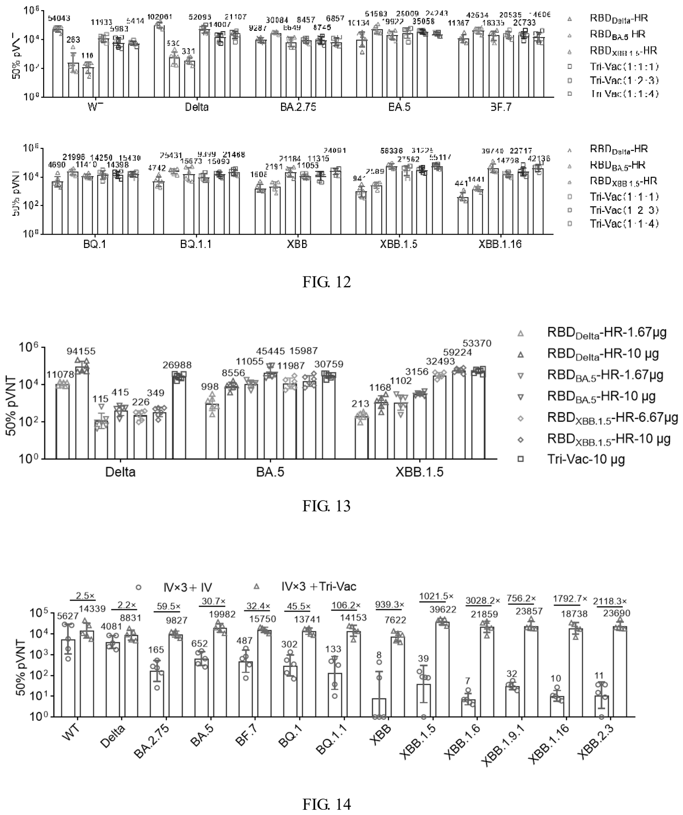

RBD_{Delta}-HR, RBD_{BA.5}-HR and RBD_{XBB.1.5}-HR proteins were used to constitute the monovalent vaccine and Tri-Vac trivalent vaccine (with different mixing ratios) to immunize mice, and sera were collected to measure the neutralizing antibody levels against WT, Delta, BA.2.75, BA.5, BF.7, BQ.1, BQ.1.1, XBB, XBB.1.5 and XBB.1.16 viruses. As shown in FIG. 12, the pure RBD_{Delta}-HR protein vaccine had strong protection against the original strain and the Delta variant strain virus, but had weak protection against the Omicron variant strain virus; the pure RBD_{BA.5}-HR only had strong protection against the BA.2.75, BA.5, BF.7, BQ.1 and BQ.1.1 variant strain virus, but had weak protection against other viruses, particularly the currently popular XBB virus and subtype thereof; the individual RBD_{XBB.1.5} vaccine has weak protection against the original strain and Delta variant strain virus, but had strong protection against the Omicron variant strain viru; however, no matter how large the mixing ratio is, the Tri-Vac trivalent vaccine (different mixing ratio) had strong protection against the original strain, Delta strain and Omicron variant strain virus, showing an obvious protection effect on XBB and the subtype thereof, in the Tri-Vac trivalent vaccine, RBD_{Delta}-HR, RBD_{BA.5}-HR and RBD_{XBB.1.5}-HR had better effect when the mixing ratio was 1:1:4. To verify the protection effect of the trivalent vaccine with a ratio of 1:1:4, as shown in FIG. 13, compared to the single component, the trivalent vaccine had strong protection against Delta, BA.5 and XBB.1.5 variant virus, and was obviously stronger than the equal dose 10µg of individual RBD_{XBB.1.5}-HR protein vaccine.

To verify the sequential immunization effect of Tri-Vac (with a ratio of 1:1:4), firstly, each mouse was subjected to intramuscular immunization of 25U inactivated vaccine (IV, produced by China National Pharmaceutical Group Co., Ltd.) for three times at days 0, 14, 42, the reinforcement for the inactivated vaccine and the sequential reinforcement for the recombinant protein vaccine were separately carried out at the 70^{th} day after the last immunization, the intramuscular injection was performed, the immunization dose was 10ug per mouse, and serums were collected at the 14^{th} day after reinforcement to detect the neutralizing antibodies of pseudovirus. As shown in FIG. 14, homologous booster immunization only provided protection against WT and Delta variant viruses, and had no protection or weak protection against Omicron variant strain virus, particularly the currently popular XBB and the subtype variant strain viruses; however, the Tri-Vac trivalent vaccine with a mixing ratio of 1:1:4 had broad-spectrum protective efficacy on all SARS-CoV-2 and had the neutralizing antibodies reaching 1000 or above.

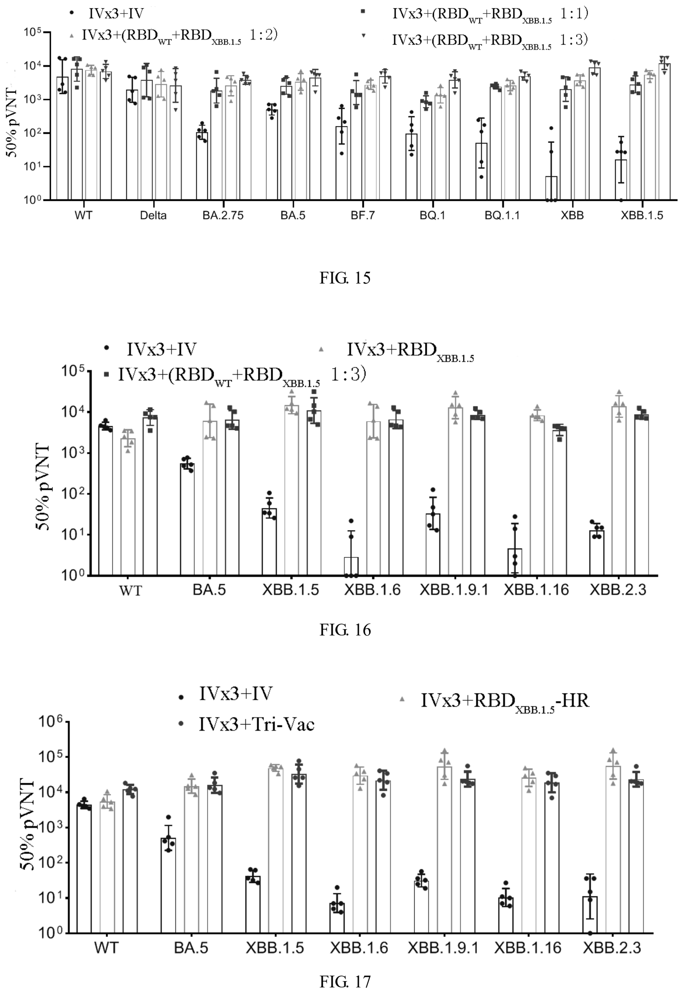

Similarly, the bivalent vaccine (RBD_{WT}+RBD_{XBB.1.5}) composed in different ratios referred to the following immunization procedure: NIH mice were immunized with the inactivated vaccines for three times at days 0, 14 and 42, the bivalent protein vaccine with different ratios was sequentially immunized on day 128 after the immunization, and serums were collected on day 14 after the sequential immunization (day 142 of the immunization procedure) to detect the neutralizing antibodies of the pseudovirus; and the immunization dose was 10µg protein per mouse. As shown in FIG. 15, the sequential immunization also showed the broad-spectrum protective efficacy on the original strain WT, Delta variant strain and Omicron variant strain virus including XBB and the subtype thereof, and however the immunization of the homologous inactivated vaccine could only provide the protection against the original strain WT and Delta variant strain virus.

As shown in FIGS. 16 and 17, according to the immunization procedure, the NIH mouse was subjected to intramuscular immunization of 25U inactivated vaccine for three times at days 0, 14, 42, the sequential reinforcement for the inactivated vaccine, RBD_{XBB.1.5}/RBD _{XBB.1.5}-HR, RBD_{WT}+RBD_{XBB.1.5} bivalent vaccine (with a mixing ratio of 1:3)/RBD_{Delta}-HR+RBD_{BA.5}-HR+RBD_{XBB.1.5}-HR trivalent vaccine (with a mixing ratio of 1:1:4) at the 84^{th} day after the last immunization, the intramuscular injection was performed, the immunization dose was 10ug per mouse; the neutralizing antibody result of pseudovirus showed the broad-spectrum protective efficacy of the bivalent vaccine and the trivalent vaccine on the original strain WT, BA.5 Omicron variant strain and Omicron variant strain virus including XBB and the subtype thereof, while the immunization of the homologous inactivated vaccine could only provide the protection against the original strain WT variant strain virus. The protective efficacy of the monovalent vaccine was equivalent to that of the bivalent vaccine.

### Embodiment 5: euvirus neutralization test for SARS-CoV-2

The neutralizing antibodies for the live variant SARS-CoV-2 in the sequential mice serum sample from the trivalent vaccine Tri-Vac (mixing at a ratio of 1:1:4) were performed through the real virus neutralization test. Each group of diluted serum was mixed with the live SARS-CoV-2 virus at a 50% tissue culture infection dose (TCID50). After incubation was performed for one hour at 37°C, the mixture was added to a 96-well microwell plate covered with Vero E6 cells (5×10⁴/well) to be incubated for 72 hours. Cell pathogenic effect (CPE) was measured with a microscope, and the titer of the neutralizing antibodies causing the EC50 (50% neutralization) to be inhibited in the immune serum was calculated.

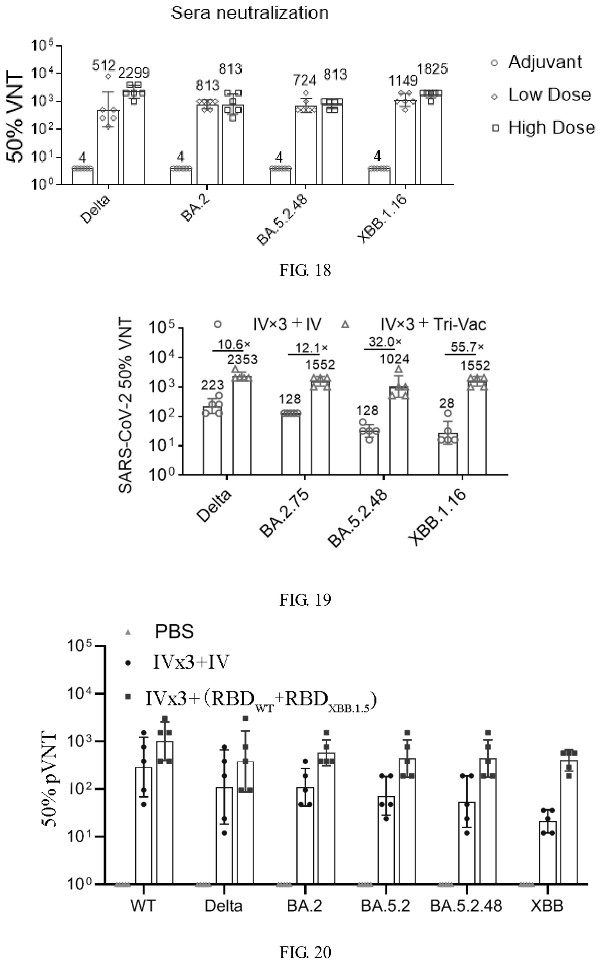

Three doses of low-dose or high-dose Tri-Vac vaccine (with a mixing ratio of 1:1:4) were muscularly immunized for three times at day 0, 14 and 28, blood was taken on the 7^{th} day after three immunizations, the neutralizing capacity for the live virus in the serum was detected, and the result was as shown in FIG. 18, the mice serum immunized with the trivalent vaccine had the stronger neutralizing capacity for the Delta, BA.2, BA.5.2.48 and XBB.1.16 live viruses than that in the control group, particularly the titer of the neutralizing antibody of the currently popular strain XBB.1.16 reached 1000 or above, and the titer in the high-dose group was higher than that in the low-dose group, showing a dose dependence.

Each mouse was subjected to intramuscular immunization of 25U inactivated vaccine for three times at days 0, 14, 42, the sequential reinforcement for the inactivated vaccine and Tri-Vac (mixed at a ratio of 1:1:4) was carried out on day 84 after the last immunization, intramuscular injection was carried out, the immunization dose was 10ug per mouse, and serums were collected at the 14^{th} day after reinforcement to detect the neutralizing antibodies of euvirus. The result was as shown in FIG. 19 that the neutralizing capacity of the trivalent vaccine Tri-Vac sequential serum to Delta, BA.2.75, BA.5.2.48 and XBB.1.16 live viruses was obviously stronger than that of the inactivated vaccine sequence, and particularly the titer of the neutralizing antibody of the currently popular strain XBB.1.16 reached 1000 or above.

Mice were immunized with inactivated vaccine three times on days 0, 14, and 42, followed by sequential immunization with either inactivated vaccine or bivalent vaccine (RBD_{WT} + RBD_{XBB.1.5}, mixed in a 1:3 ratio) on day 128, blood was collected 7 days after the fourth immunization for live virus neutralization assays, immunization dose: 10 µg protein per mouse, serum was collected 14 days after the booster for live virus neutralizing antibody detection. The result was as shown in FIG. 20 that the neutralizing capacity of the bivalent vaccine sequential serum to wild, Delta, BA.2, BA.5.2, BA.5.2.48 and XBB live viruses was obviously stronger than that of the inactivated vaccine sequence, and particularly the titer of the neutralizing antibody of the currently popular strain XBB.1.16 reached 1000 or above.

### Embodiment 6: attack by SARS-CoV-2 XBB.1.16 variant

BALB/c mouse was muscularly immunized with low-dose (5µg) and high-dose (10µg) trivalent vaccine Tri-Vac (mixing at a ratio of 1:1:4) on day 0, 28 and 56. The mouse immunized with the separate adjuvant served as the control group, and n=6 mice were in each group. Then, all mice were subjected to intranasal challenge with live SARS-CoV-2 XBB.1.16 Omicron variant (1×10⁶ PFU) on day 21 after the last vaccination. The mice were subjected to euthanasia and their tissues were collected on day 4 after infection. Pathological changes of lung tissues were observed through hematoxylin eosin stain. The viral loads in turbinate, broncho and lung tissue samples were detected by observing the mouse weight and measuring the reverse transcription quantitative polymerase chain reaction (RT-qPCR) of virus genome (gRNA), the primer sequence was 5'-GACCCCAAAATCAGCGAAAT-3' (forward) (SEQ ID No.40), 5'-TCTGGTTACTGCCCAGTTGAATCTG-3'(reverse) (SEQ ID No.41), and the probe sequence was 5'-FAM-ACGCCGCATTACGTTTGGTGGGACC-BHQ1-3' (SEQ ID No.42). All procedures related to SARS-CoV-2 Omicron variant mouse attack were examined and approved by Institutional Animal Care and Use Committee of Institute of Medical Biology,Chinese Academy of Medical Sciences, and were carried out in ABSL-4 facilities of Kunming National High-level Biosafety Primate Research Center.

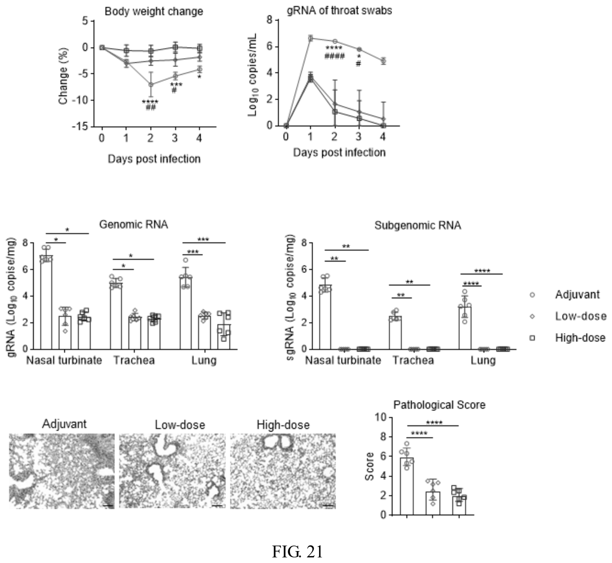

As shown in FIG. 21, after virus infection, the mouse weight change in the trivalent vaccine Tri-Vac group mixed at a ratio of 1:1:4 was obviously weaker than the mouse weight in the adjuvant control group; the throat swab virus of the mouse in the trivalent vaccine Tri-Vac treatment group was basically cleared up, the viruses RNA in the turbinate, trachea and lung tissues were not detected, the lung histology was normal, and the alveolar structure was complete, without obvious inflammation; however, in the adjuvant control group, the throat swab sample still had more virus loads, the virus RNA level in the turbinate, trachea and lung tissues were still high, the serious pathological change might be observed through pathological section, including a multifocal consolidation area, alveolar septum thickening and alveolar congestion; in addition, small inflammatory lesion composed of macrophage, neutrophil and lymphocyte were occasionally observed around small vessels. It has showed that the trivalent vaccine Tri-Vac mixed at a ratio of 1:1:4 had a complete protection against infection by XBB.1.16 Omicron variant strain and had better safety.

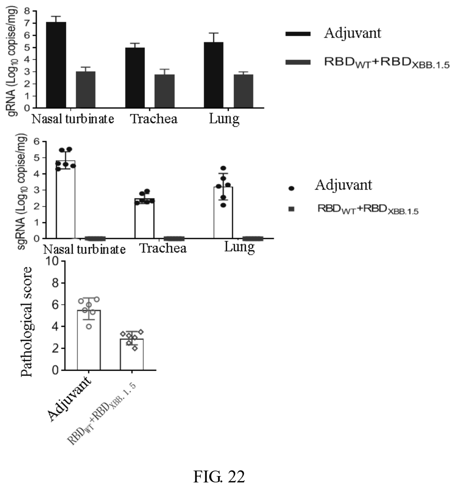

As shown in FIG. 22, the viruses RNA in the turbinate, trachea and lung tissues were not detected in the mouse of the sequential treatment group of the bivalent vaccine (RBD_{WT}+ RBD_{XBB.1.5}) mixed at a ratio of 1:3, and basically cleared up; however, the virus RNA level in the turbinate, trachea and lung tissues were high in the adjuvant control group; the pathological tissue scoring was also obviously lower than that of the control group, showing that the bivalent vaccine (RBD_{WT}+ RBD_{XBB.1.5}) mixed at a ratio of 1:3 in the sequential immunization had a complete protection against infection by XBB.1.16 Omicron variant strain and had better safety.

## Claims

1. A protein against infection by a SARS-CoV-2 Omicron variant strain and a subtype thereof, wherein: comprising an amino acid sequence as shown in any one of SEQ ID No.1-SEQ ID No.6; or an amino acid sequence that has a homology of over 98% and the same or similar biological activity with the amino acid sequence as shown in any one of SEQ ID No.1-SEQ ID No.6.

2. The protein according to claim 1, wherein the homologous amino acid sequence as shown in SEQ ID No.1, SEQ ID No.2, or SEQ ID No.3 meets at least one of the following items: in SEQ ID No.1, SEQ ID No.2, or SEQ ID No.3, the 52nd amino acid F is mutated into S; the 54th amino acid P is mutated into S; and the 56th amino acid F is mutated into S.

3. The protein according to claim 1, wherein the homologous amino acid sequence as shown in SEQ ID No.4 or SEQ ID No.5 meets at least one of the following items: in SEQ ID No.4 or SEQ ID No.5, the 351th amino acid sequence F is mutated into S; the 353th amino acid sequence P is mutated into S; and the 355th amino acid sequence F is mutated into S; and the homologous amino acid sequence as shown in SEQ ID No.6 meets at least one of the following items: in SEQ ID No.6, the 352th amino acid sequence F is mutated into S; the 354th amino acid sequence P is mutated into S; and the 356th amino acid sequence F is mutated into S.

4. The protein according to any one of claims 1 to 3, wherein the amino acid sequence of the protein is selected from at least one of SEQ ID No.1-SEQ ID No.12.

5. A precursor for the protein according to any one of claims 1 to 4, wherein a signal peptide and/or a protein tag is linked to the protein against the infection by the SARS-CoV-2 Omicron variant strain and the subtype thereof; and preferably, the protein tag is selected from at least one of the following: a histidine tag, a thioredoxin tag, a glutathione transferase tag, a ubiquitin-like protein modification tag, a maltose-binding protein tag, a c-Myc protein tag, an Avi tag protein tag, and a nitrogen source using substance A protein tag.

6. The precursor according to claim 5, wherein a protease recognition region for excising the protein tag is also linked to the protein against the infection by the SARS-CoV-2 Omicron variant strain and the subtype thereof; and preferably, the protease is selected from at least one of the following: enterokinase, TEV protease, thrombin, a blood coagulation factor Xa, carboxypeptidase A, and rhinovirus 3c protease.

7. The precursor according to claim 5 or 6, wherein an amino acid sequence of the precursor is selected from at least one of SEQ ID No.13-SEQ ID No.24.

8. A polynucleotide, wherein: encoding the protein according to any one of claims 1 to 4 or the precursor according to any one of claims 5 to 7.

9. The polynucleotide according to claim 8, wherein a nucleotide sequence is selected from at least one of SEQ ID No.25-SEQ ID No.30.

10. A recombinant vector, wherein: comprising the polynucleotide according to claim 8 or 9.

11. The recombinant vector according to claim 10, wherein at least one of an insect baculovirus expression vector, a mammalian cell expression vector, an escherichia coli expression vector and a yeast expression vector is used; preferably, the insect baculovirus expression vector is pFastBac1; preferably, the escherichia coli expression vector is pET32a; preferably, the yeast expression vector is pPICZaA; preferably, the mammalian cell expression vector is a CHO cell expression vector; and further preferably, the CHO cell expression vector is pTTS or FTP-002.

12. A host cell, wherein: comprising the recombinant vector according to claim 10 or 11.

13. The host cell according to claim 12, wherein at least one of an insect cell, a mammalian cell, escherichia coli and a yeast is used; preferably, the insect cell is selected from at least one of a sf9 cell, a sf21 cell and a Hi5 cell; and preferably, the mammalian cell is a CHO cell.

14. A preparation method for the protein according to any one of claims 1 to 4 or the precursor according to any one of claims 5 to 7, wherein: comprising the following steps: culturing the host cell according to claim 12 or 13, such that the host cell expresses the protein or the precursor, then recovering the protein, thereby obtaining the protein.

15. A protein composition against infection by SARS-CoV-2 or a mutant thereof, wherein: comprising a combination of at least two or more of a Delta protein, a Delta full-length S protein, a BA.5 protein, a BQ.1.1 protein, an XBB.1.5 protein, a BA.4/5 full-length S protein, a BQ.1.1 full-length S protein, an XBB.1.5 full-length S protein, a Mu-BA.5 protein, a Mu-BQ.1.1 protein, a Mu-XBB.1.5 protein, a full-length S protein after BA.4/5 amino acid mutation, a full-length S protein after BQ.1.1 amino acid mutation and a full-length S protein after XBB.1.5 amino acid mutation; and preferably, the combinations of at least 2-4 proteins.

16. The composition according to claim 15, wherein: comprising a combination of at least two or more of the BA.5 protein, the BQ.1.1 protein, the XBB.1.5 protein, the BA.4/5 full-length S protein, the BQ.1.1 full-length S protein and the XBB.1.5 full-length S protein.

17. The composition according to claim 15, wherein: comprising a combination of at least two or more of the Delta protein, the Mu-BA.5 protein, the Mu-BQ.1.1 protein and the Mu-XBB.1.5 protein.

18. The composition according to claim 15, wherein: comprising a combination of at least two or more of the Mu-BA.5 protein, the Mu-BQ.1.1 protein, the Mu-XBB.1.5 protein, the full-length S protein after the BA.4/5 amino acid mutation, the full-length S protein after the BQ.1.1 amino acid mutation and the full-length S protein after the XBB.1.5 amino acid mutation.

19. The composition according to claim 15, wherein the BA.5 protein, the BQ.1.1 protein, the XBB.1.5 protein, the BA.4/5 full-length S protein, the BQ.1.1 full-length S protein, the XBB.1.5 full-length S protein, the Mu-BA.5 protein, the Mu-BQ.1.1 protein, the Mu-XBB.1.5 protein, the full-length S protein after the BA.4/5 amino acid mutation, the full-length S protein after the BQ.1.1 amino acid mutation and the full-length S protein after the XBB.1.5 amino acid mutation comprise the protein according to any one of claims 1 to 4 or the precursor according to any one of claims 5 to 7.

20. The composition according to claim 19, wherein amino acid sequences of the Delta protein, the Delta full-length S protein, the BA.5 protein, the BQ.1.1 protein, the XBB.1.5 protein, the BA.4/5 full-length S protein, the BQ.1.1 full-length S protein, the XBB.1.5 full-length S protein, the Mu-BA.5 protein, the Mu-BQ.1.1 protein, the Mu-XBB.1.5 protein, the full-length S protein after the BA.4/5 amino acid mutation, the full-length S protein after the BQ.1.1 amino acid mutation and the full-length S protein after the XBB.1.5 amino acid mutation are constructed based on at least one amino acid sequence as shown in SEQ ID No.31, SEQ ID No.33 and SEQ ID No.1-SEQ ID No.24.

21. A vaccine for preventing and/or treating infection by a SARS-CoV-2 Omicron variant and a subtype thereof, wherein: comprising the protein according to any one of claims 1 to 4, the precursor according to any one of claims 5 to 7 and/or the protein composition according to any one of claims 15 to 20, as well as a pharmaceutically acceptable excipient or adjuvant component.

22. The vaccine according to claim 21, wherein the adjuvant component is an immunologic adjuvant; and preferably, the immunologic adjuvant is selected from at least one of the following: squalene-based oil-in-water emulsion, aluminum salt, calcium salt, phytosaponin, phytopolysaccharide, monophosphate lipid A, muramyl dipeptide, muramyl tripeptide, bacterial toxin, GM-CSF cytokine, lipid and a cationic liposomal material.

23. The vaccine according to claim 22, wherein the vaccine meets at least one of the following: the squalene-based oil-in-water emulsion is MF59; the aluminum salt is selected from at least one of aluminum hydroxide and alum; the calcium salt is tricalcium phosphate; the phytosaponin is either QS-21 or ISCOM; the phytopolysaccharide is astragalus polysaccharide; the bacterial toxin is selected from at least one of recombinant cholera toxin and diphtheria toxin; the lipid is selected from at least one of the following: phosphatidylethanolamine, phosphatidylcholine, cholesterol, dioleoylphosphatidylethanolamine; the cationic liposome material is selected from at least one of the following: (2,3-dioleoyloxypropyl) trimethyl ammonium chloride, N-[1-(2,3-dioleoyl chloride) propyl]-N,N,N-trimethylamine chloride, cationic cholesterol, dimethyl-2,3-dioleyloxypropyl-2-(2-spermidinecarboxamido)ethyl ammonium trifluoroacetate, trimethyldodecylammonium bromide, trimethyltetradecylammonium bromide, trimethylhexadecylammonium bromide, dimethyldioctadecylammonium bromide, and CpG ODN.

24. The vaccine according to any one of claims 20 to 23, wherein the vaccine is an injection preparation, a nasal spray preparation and an oral preparation; and preferably, the vaccine is an intramuscular injection preparation.

25. Use of the protein according to any one of claims 1 to 4, the precursor according to any one of claims 5 to 7 and/or the protein composition according to any one of claims 15 to 30 or the vaccine according to any one of claims 21 to 24 in preparing drugs for treating and/or preventing SARS-CoV-2 or mutant infection or pathogenicity thereof.

26. The use according to claim 25, wherein the SARS-CoV-2 mutant is selected from at least one of Alpha, Beta, Gamma, Delta or Omicron strains, and the Omicron strain comprises at least one of BA.1, BA.2, BA.2.12.1, BA.4, BA.5, BQ.1.1 and XBB.1.5.

27. A mRNA vaccine for preventing and/or treating infection by a SARS-CoV-2 Omicron variant strain and a subtype thereof, wherein: comprising a polynucleotide sequence according to claim 8.

28. An adenovirus vaccine for preventing and/or treating infection by a SARS-CoV-2 Omicron variant strain and a subtype thereof, wherein: comprising a polynucleotide sequence according to claim 8.
